# EUROPÄISCHE PATENTSCHRIFT

(11) **EP 1 519 934 B1**
(45) Veröffentlichungstag und Bekanntmachung des Hinweises auf die Patenterteilung: **19.09.2007**
(21) Anmeldenummer: 03762498.8
(22) Anmeldetag: 20.06.2003
(51) Int. Cl.: C07D 471/04, A61K 31/47

(54) **PYRAZOLOISOQUINOLINENDERIVATEN ZUR INHIBIERUNG VON NFKAPPAB-INDUZIERENDE KINASE**
PYRAZOLOISOQUINOLINE DERIVATIVES FOR INHIBITING NF$G(K)B-INDUCING KINASE (NIK)
DERIVES DE PYRAZOLOISOQUINOLINE PERMETTANT D'INHIBER UNE KINASE INDUISANT NF$G(K)B (NIK)

(30) Priorität: 03.07.2002 DE 10229762
(43) Veröffentlichungstag der Anmeldung: 06.04.2005
(73) Patentinhaber: Sanofi-Aventis Deutschland GmbH, 65929 Frankfurt am Main (DE)
(72) Erfinder: FLOHR, Stefanie, CH-4153 Reinach (CH); NAUMANN, Thorsten, 65926 Frankfurt am Main (DE)
(86) Internationale Anmeldenummer: PCT/EP2003/006500
(87) Internationale Veröffentlichungsnummer: WO 2004/005287

(56) Entgegenhaltungen:
- EP-A- 1 134 221
- WO-A-02/44153
- GB-A- 2 185 255
- YAMAMOTO YUMI ET AL: "Role of the NF-kappaB pathway in the pathogenesis of human disease states" CURRENT MOLECULAR MEDICINE, BENTHAM SCIENCE PUBLISHERS, GB, Bd. 1, Nr. 3, Juli 2001 (2001-07), Seiten 287-296, XP001094465 ISSN: 1566-5240

## Beschreibung

Die Erfindung betrifft neue Pyrazoloisoquinolinenderivate, Verfahren zu ihrer Herstellung und Verwendung derselben als Arzneimittel, insbesondere deren Verwendung zur Inhibierung von NFκB-induzierende Kinase (NIK). Die Pyrazoloisoquinolinenderivate können daher zur Behandlung degenerativer Gelenkerkrankungen eingesetzt werden.

NFκB ist ein heterodimerer Transkriptionsfaktor, der eine Vielzahl von Gene aktivieren kann, die unter anderen für proinflammatorische Cytokine wie IL-1, IL-2, TNFα oder IL-6 kodieren. NFκB liegt im Cytosol von Zellen komplexiert mit seinem natürlich vorkommenden Inhibitor IκB vor. Die Stimulation von Zellen, beispielsweise durch Cytokine, führt zur Phosphorylierung und anschließenden proteolytischen Abbau von IκB. Dieser proteolytische Abbau führt zur Aktivierung von NFκB, das anschließend in den Kern der Zelle wandert und dort eine Vielzahl von proinflammatorischen Genen aktiviert.
In Erkrankungen wie Rheumatoider Arthritis (bei der Entzündung), Osteoarthritis oder Asthma ist NFκB über das normale Maß hinaus aktiviert. Es konnte gezeigt werden, dass Arzneimittel wie Glucocorticoide, Salicylate oder Goldsalze, die in der Rheumatherapie eingesetzt werden, an verschiedenen Stellen in die NFκBaktivierende Signalkette inhibierend eingreifen oder direkt mit der Transkription der Gene interferieren.

Die IkB Kinase (IKK) hat eine zentrale Funktion im NFkB Signaltransduktionsweg da sie die Phosphorylierung von IkB vermittelt. IKK wird ebenfalls durch Phosphorylierung aktiviert. Die NFkB-induzierende Kinase (NIK) ist eine Ser/Thr Kinase und an der Aktivierung von IKK beteiligt. Durch Überexpression von NIK in Zellkultur konnte Stimulus-unabhängig die Expression von NFkB-aktivierten Reportergenen oder die Expression des NFkB-induzierte Adhesionsmoleküls ICAM1 verstärkt werden. NIK vermittelt diesen Effekt durch Interaktion und Phosphorylierung der IKKα Untereinheit von IKK. Im Gegensatz dazu konnte durch die Überexpression einer dominant negativen NIK Mutante in Zellkultur die Expression eines NFkB-aktivierten Reportergens als auch die IL1-induzierte Expression des Adhesionsmoleküls ICAM1 gehemmt werden. Durch die Überexpression der C-terminalen Domäne von NIK, die für die Interaktion mit IKK verantwortlich ist, konnte in Zellkultur die TNFα-induzierte Expression eines NFkB-aktivierten Reportergenes inhibiert werden. Pyrazoloisoquinolinenderivate mit inhibitorischer Aktivität gegen NIK können ebenfalls die TNFα-Freisetzung in LPS- und IL1 β-stimulierten humanen peripheren Blutlymphozyten, sowie die Freisetzung von IL1 β, TNFα und 1L6 in LPS-stimuliertem humanem Vollblut hemmen.
Pyrazol-isoquinolineverbindungen mit anti-inflammatorischer Aktivität wurden bereits in der Veröffenlichungsschrift GB 2 185 255 A beschrieben. Substituierte Beta-Carbolin-Derivate werden in EP 1134221 A1 beschrieben und deren Verwendung zur Behandlung von Erkrankungen, in deren Verlauf eine erhöhte Aktivität von NFkB vorkommt.

In dem Bestreben wirksame Verbindungen zur Behandlung von Erkrankungen zu erhalten an deren Verlauf eine verstärkte Aktivität von NFkB-induzierende Kinase beteiligt ist wurde nun gefunden, dass die erfindungsgemäßen Pyrazoloisoquinolinenderivate starke und sehr spezifische Inhibitoren von NIK sind und eine gute Wasserlöslichkeit aufweisen.

Die Erfindung betrifft daher die Verbindungen der Formel I 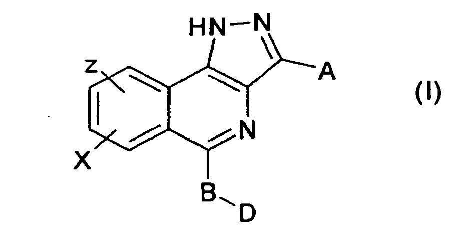 und/oder eine stereoisomere Form der Verbindung der Formel I und/oder ein physiologisch verträgliches Salz der Verbindung der Formel I, wobei
A für
   1. -(C₁-C₆)-Alkyl, worin Alkyl gerade oder verzweigt ist und ein- oder zweifach unabhängig voneinander substituiert ist durch
      1.1 -O-R1 oder
      1.2 C(O)-OR1, worin R1 für
         a) Wasserstoffatom oder
         b) -(C₁-C₆)-Alkyl steht,
      2. -O-R1,
      3. C(O)-OR1,
      4. Heteroaryl mit 5 bis 14 Ringgliedern, worin Heteroaryl unsubstituiert oder ein-, zwei- oder dreifach unabhängig voneinander durch R2 substituiert ist, steht, oder
      5. -(C₁-C₆)-Alkyl, worin Alkyl gerade oder verzweigt ist,
B für
   1. eine kovalente Bindung oder
   2. -(C₁-C₄)-Alkylen, worin Alkylen gerade oder verzweigt ist und ein- oder
      zweifach unabhängig voneinander durch R1 substituiert ist und R1 wie oben definiert ist, steht,
D für
   1. Heteroaryl mit 5 bis 14 Ringgliedern, worin Heteroaryl unsubstituiert
      oder ein-, zwei- oder dreifach unabhängig voneinander substituiert ist durch R2,
      worin R2 für
      a) Wasserstoffatom,
      b) -(C₁-C₄)-Alkyl,
      c) -OH,
      d) -O-(C₁-C₄)-Alkyl,
      e) Halogen oder
      f) -N(R3)-R4, worin R3 und R4 unabhängig voneinander Wasserstoffatom oder -(C₁-C₄)-Alkyl bedeuten, steht,
   2. Heterocyclus mit 5 bis 12 Ringgliedern, worin Heterocyclus unsubstituiert oder ein-, zwei- oder dreifach unabhängig voneinander substituiert ist durch R2, und R2 wie oben definiert ist,
   3. -(C₆-C₁₄)-Aryl, worin Aryl ein-, zwei- oder dreifach unabhängig voneinander substituiert ist durch
      a) -OH,
      b) -O-(C₁-C₄)-Alkyl, oder
      c) -N(R3)-R4, worin R3 und R4 unabhängig voneinander Wasserstoffatom oder -(C₁-C₄)-Alkyl bedeuten, oder
      4. -(C₃-C₆)-Cycloalkyl, worin Cycloalkyl unsubstituiert oder ein-, zwei- oder dreifach unabhängig voneinander substituiert ist durch R2, und R2 wie oben definiert ist, steht, und
   X und Z gleich oder verschieden sind und unabhängig voneinander für
      a) Wasserstoffatom,
      b) -(C₁-C₄)-Alkyl,
      c) -OH,
      d) -O-(C₁-C₄)-Alkyl oder
      e) Halogen stehen.

Die Erfindung betrifft ferner Verbindungen der Formel I, wobei
A für
   1. -(C₁-C₃)-Alkyl, worin Alkyl gerade oder verzweigt ist und ein- oder zweifach unabhängig voneinander substituiert ist durch
      1.1 -O-R1 oder
      1.2 -C(O)-OR1, worin R1 für
         a) Wasserstoffatom oder
         b) -(C₁-C₃)-Alkyl steht, oder
   2. C(O)-OR1, steht,
B für eine kovalente Bindung steht,
D für
   1. Phenyl, worin Phenyl unsubstituiert oder ein-, zwei- oder dreifach unabhängig voneinander substituiert ist durch R2,
      worin R2 für
      a) Wasserstoffatom,
      b) -(C₁-C₄)-Alkyl oder
      c) -N(R3)-R4, worin R3 und R4 unabhängig voneinander Wasserstoffatom oder -(C₁-C₃)-Alkyl bedeuten, steht,
   2. Pyridyl, worin Pyridyl unsubstituiert oder ein-, zwei- oder dreifach unabhängig voneinander substituiert ist durch R2 und R2 wie oben definiert ist, oder
   3. -(C₄-C₆)-Cycloalkyl, worin Cycloalkyl unsubstituiert oder ein-, zwei- oder dreifach unabhängig voneinander substituiert ist durch R2 und R2 wie oben definiert ist, steht, und
X und Z gleich oder verschieden sind und unabhängig voneinander für Wasserstoffatom oder Halogen stehen.

Die Erfindung betrifft ferner Verbindungen der Formel I ausgewählt aus der Gruppe
5-(3-Methoxy-phenyl)-3-methyl-1 H-pyrazolo[4,3-c]isoquinolin,
3-(3-Methyl-1 H-pyrazolo[4,3-c]isoquinolin-5-yl)-phenol,
5-(2-Methoxy-phenyl)-3-methyl-1 H-pyrazol[4,3-c]isoquinolin,
5-(2,3-Dimethoxy-phenyl)-3-methyl-1 H-pyrazol[4,3-c]isoquinolin,
5-(2,4-Dimethoxy-phenyl)-3-methyl-1 H-pyrazol[4,3-c]isoquinolin,
5-(2,6-Dimethoxy-phenyl)-3-methyl-1 H-pyrazol[4,3-c]isoquinolin,
5-(3,4-Dimethoxy-phenyl)-3-methyl-1 H-pyrazol[4,3-c]isoquinolin,
5-(3,5-Dimethoxy-phenyl)-3-methyl-1 H-pyrazol[4,3-c]isoquinolin,
5-(2,3,4-Trimethoxy-phenyl)-3-methyl-1 H-pyrazol[4,3-c]isoquinolin,
5-(2,4,6-Trimethoxy-phenyl)-3-methyl-1 H-pyrazol[4,3-c]isoquinolin,
5-(3,4,5-Trimethoxy-phenyl)-3-methyl-1 H-pyrazol[4,3-c]isoquinolin,
5-(2-Ethoxy-phenyl)-3-methyl-1 H-pyrazol[4,3-c]isoquinolin,
5-(4-Diethylamino-phenyl)-3-methyl-1 H-pyrazol[4,3-c]isoquinolin,
3-Methyl-5-pyridin-4-yl-1 H-pyrazol[4,3-c]isoquinolin,
3-Methyl-5-pyridin-3-yl-1 H-pyrazol[4,3-c]isoquinolin,
3-Methyl-5-pyridin-2-yl-1 H-pyrazol[4,3-c]isoquinolin,
3-Methyl-5-(1-methyl-piperidin-4-yl)-1 H-pyrazol[4,3-c]isoquinolin,
7,8-Dimethoxy-5-(3-methoxy-phenyl)-3-methyl-1 H-pyrazol[4,3-c]isoquinolin,
7,8-Dimethoxy-3-methyl-5-pyridin-2-yl-1 H-pyrazol[4,3-c]isoquinolin,
7,8-Dimethoxy-3-methyl-5-pyridin-3-yl-1 H-pyrazol[4,3-c]isoquinolin,
7-Methoxy-5-(3-methoxy-phenyl)-3-methyl-1 H-pyrazol[4,3-c]isoquinolin,
2-(3-Methyl-1 H-pyrazol[4,3-c]isoquinolin-5-yl)-phenol,
4-(3-Methyl-1 H-pyrazol[4,3-c]isoquinolin-5-yl)-benzen-2,4-diol oder
4-(3-Methyl-1 H-pyrazol[4,3-c]isoquinolin-5-yl)-benzen-1,2-diol.

Unter dem Begriff "(C₁-C₆)-Alkyl" werden Kohlenwasserstoffreste verstanden, deren Kohlenstoffkette geradkettig oder verzweigt ist und 1 bis 6 Kohlenstoffatome enthält, beispielsweise Methyl, Ethyl, Propyl, IsoPentyl, Neopentyl, Hexyl, 2,3-Dimethylbutan oder Neohexyl. "(C₃-C₆)-Cycloalkyl-Reste" sind beispielsweise Verbindungen, die sich von 3- bis 6-gliedrige Monocyclen wie Cyclopropyl, Cyclobutyl, Cyclopentyl oder Cyclohexyl herleiten. Unter dem Begriff "Halogen" wird Fluor, Chlor, Brom oder Jod verstanden.
Unter dem Begriff "(C₆-C₁₄)-Aryl" werden aromatische Kohlenstoffreste verstanden mit 6 bis 14 Kohlenstoffatomen im Ring. (C₆-C₁₄)-Arylreste sind beispielsweise Phenyl, Naphthyl, zum Beispiel 1-Naphthyl, 2-Naphthyl, Biphenylyl, zum Beispiel 2-Biphenylyl, 3-Biphenylyl und 4-Biphenylyl, Anthryl oder Fluorenyl. Biphenylylreste, Naphthylreste und insbesondere Phenylreste sind bevorzugte Arylreste.

In monosubstituierten Phenylresten kann sich der Substituent in der 2-Position, der 3-Position oder der 4-Position befinden. Zweifach substituiertes Phenyl kann in der 2,3-Position, der 2,4-Position, der 2,5-Position, der 2,6-Position, der 3,4-Position oder der 3,5-Position substituiert sein. in dreifach substituierten Phenylresten können sich die Substituenten in der 2,3,4-Position, der 2,3,5-Position, der 2,4,5-Position, der 2,4,6-Position, der 2,3,6-Position oder der 3,4,5-Position befinden.

Der Begriff "Heteroaryl mit 5 bis 14 Ringgliedern" steht für einen Rest eines monocyclischen oder polycyclischen aromatischen Systems mit 5 bis 14 Ringgliedern, das 1, 2, 3, 4 oder 5 Heteroatome als Ringglieder enthält. Beispiele für Heteroatome sind N, 0 und S. Sind mehrere Heteroatome enthalten, können diese gleich oder verschieden sein.
Besonders bevorzugt steht Heteroaryl für einen monocyclischen oder bicyclischen aromatischen Rest mit 5 bis 10 Ringgliedern, insbesondere für einen 5-gliedrigen bis 6-gliedrigen monocyclischen aromatischen Rest, der 1, 2 oder 3, insbesondere 1 oder 2, gleiche oder verschiedene Heteroatome aus der Reihe N, 0 und S enthält.
Der Begriff "Heterocyclus mit 5 bis 12 Ringgliedern" steht für einen monocyclischen oder bicyclischen 5-gliedrigen bis 12-gliedrigen heterocyclischen Ring, der teilweise gesättigt oder vollständig gesättigt ist. Beispiele für Heteroatome sind N, 0 und S.

Beispiele für die Begriffe "Heteroaryl mit 5 bis 14 Ringgliedern" oder "Heterocyclus mit 5 bis 12 Ringgliedern" sind Reste wie Acridinyl, Azaindole (1H-pyrrolopyridinyl), Azabenzimidazoyl, Azaspirodecanyl, Azepinyl, Azetidinyl, Aziridinyl, Benzimidazolyl, Benzofuranyl, Benzothiofuranyl, Benzothiophenyl, Benzoxazolyl, Benzthiazolyl, Benztriazolyl, Benztetrazolyl, Benzisoxazolyl, Benzisothiazolyl, Carbazolyl, 4aH-Carbazolyl, Carbolinyl, Chinazolin, Chinolin, Chinolidinyl, Chinoxalinyl; Chromanyl, Chromenyl, Cinnolinyl, Decahydrochinolinyl, 4,5-Dihydrooxazolinyl, Dioxazolyl, Dioxazinyl, 1,3-Dioxolanyl, 1,3-Dioxolenyl, 6H-1,5,2-Dithiazinyl, Dihydrofuro[2,3-b]-tetrahydrofuranyl, Furanyl, Furazanyl, Imidazolidinyl, Imidazolinyl, imidazolyl, 1H-Indazolyl, Indolinyl, Indolizinyl, Indolyl, 3H-Indolyl, Isobenzofuranyl, Isochinolin, Isochromanyl, Isoindazolyl, Isoindolinyl, Isoindolyl, Isoquinolinyl, isothiazolyl, Isothiazolidinyl, Isothiazolinyl, Isoxazolyl, Isoxazolinyl, Isoxazolidinyl, 2-Isoxazolinyl, Ketopiperazinyl, Morpholinyl, Naphthyridinyl, Octahydroisoquinolinyl, Oxadiazolyl, 1,2,3-Oxadiazolyl, 1,2,4-Oxadiazolyl, 1,2,5-Oxadiazolyl, 1,3,4-Oxadiazolyl, 1,2-Oxa-thiepanyl, 1,2-Oxathiolanyl, 1,4-Oxazepanyl, 1,2-Oxazinyl, 1,3-Oxazinyl, 1,4-Oxazinyl, Oxazolidinyl, Oxazolinyl, Oxazolyl, Oxetanyl, Oxocanyl, Phenanthridinyl, Phenanthrolinyl, Phenazinyl, Phenothiazinyl, Phenoxathiinyl, Phenoxazinyl, Phthalazinyl, Piperazinyl, Piperidinyl, Pteridinyl, Purinyl, Pyranyl, Pyrazinyl, Pyrazolidinyl, Pyrazolinyl, Pyrazolyl, Pyridazinyl, Pyridooxazolyl, Pyridoimidazolyl, Pyridothiazolyl, Pyridinyl, Pyridyl, Pyrimidinyl, Pyrrolidinyl, Pyrrolidinonyl, Pyrrolinyl, 2H-Pyrrolyl, Pyrrolyl, Tetrahydrofuranyl, Tetrahydroisochinolinyl, Tetrahydrochinolinyl, Tetrahydrofuranyl, Tetrahydropyranyl, 1,4,5,6-Tetrahydro-pyridazinyl, Tetrahydropyridinyl, Tetrahydrothiophenyl, Tetrazinyl, Tetrazolyl, 6H-1,2,5-Thiadiazinyl, 1,2,3-Thiadiazolyl, 1,2,4-Thiadiazolyl, 1,2,5-Thiadiazolyl, 1,3,4-Thiadiazolyl, Thianthrenyl, 1,2-Thiazinyl,1,3-Thiazinyl,1,4-Thiazinyl,1,3-Thiazolyl, Thiazolyl, Thiazolidinyl, Thiazolinyl, Thienyl, Thietanyl, Thienothiazolyl, Thienooxazolyl, Thienoimidazolyl, Thietanyl, Thiomorpholinyl, Thiophenolyl, Thiophenyl, Thiopyranyl, 1,2,3-Triazinyl, 1,2,4-Triazinyl, 1,3,5-Triazinyl, 1,2,3-Triazolyl, 1,2,3-Triazolyl, 1,2,4-Triazolyl, 1,2,5-Triazolyl, 1,3,4-Triazolyl und Xanthenyl.
Bevorzugt sind Reste, die sich von Benzodioxolan, Benzofuran, Benzothiazol, Benzothiophen, Benzoxazol, β-Carbolin, Chinazolin, Chinolin, Chinoxalin, Cinnolin, Cyclohepta[b]-5-pyrrol, 4,5-Dihydro-1,3-oxazol, Dihydropyridin, 4,5-Dihydro-1,3-thiazol, 1,3-Dioxolan, Furan, 3-Hydroxypyrro-2,4-dion, Imidazol, 2-Imidazolin, Imidazolidin, Indazol, Indol, Indolin, Isochinolin, Isoindol, Isoindolin, Isoxazolone; isothiazol, Isoxazol, Morpholin, Oxadiazolidindion, Oxadiazolon, 1,2,3,5-Oxathiadiazol-2-Oxid, Oxazol, 1,3-Oxazolidin, 5-Oxo-1,2,4-Thiadiazol, Perhydroazepin, Perhydro-1,4-dioxan, Phthalazin, Piperazin, Piperidin, Pyrazin, Pyrazol, Pyridin, Pyrimidin, Pyrrol, Pyrrolidin, Pyrrolin, Tetrahydrofuran, 1,2,3,4-Tetrahydroisoquinolin, Tetrahydropyridin, 1,2,3,4-Tetrahydroquinolin, Tetrahydrothiophen, Tetrazol, 1,3-Thiazol, Thiazolidin, Thiomorpholin, Thiophen, Triazol und Triazolon ableiten lassen.

Die Erfindung betrifft ferner ein Verfahren zur Herstellung der Verbindung der Formel I und/oder einer stereoisomeren Form der Verbindung der Formel I und/oder eines physiologisch verträglichen Salzes der Verbindung der Formel I, das dadurch gekennzeichnet ist, dass man
a) eine Verbindung der Formel IV 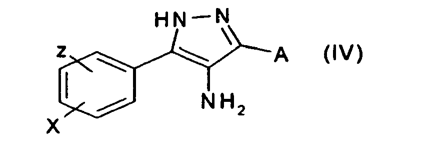 mit einer Verbindung der Formeln Va oder Vb 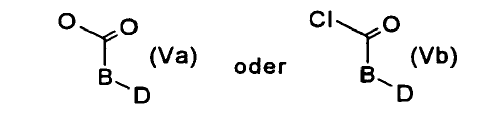 zu einer Verbindung der Formel VI umsetzt 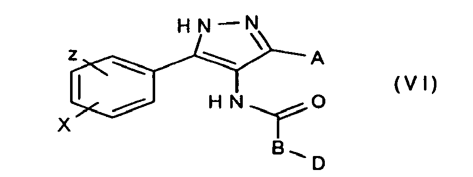 und in Anwesenheit von Phsphorpentoxid und Phosphoroxychlorid in eine geschützte Verbindung der Formel I umsetzt, und abschließend die Schutzgruppe abspaltet,
b) eine nach Verfahren a) hergestellte Verbindung der Formel 1, die aufgrund ihrer chemischen Struktur in enantiomeren Formen auftritt, durch Salzbildung mit enantiomerenreinen Säuren oder Basen, Chromatographie an chiralen Stationärphasen oder Derivatisierung mittels chiraler enantiomerenreinen Verbindungen wie Aminosäuren, Trennung der somit erhaltenen Diastereomeren, und Abspaltung der chiralen Hilfsgruppen in die reinen Enantiomeren auftrennt, oder
c) die nach den Verfahren a) oder b) hergestellte Verbindung der Formel I entweder in freier Form isoliert oder im Falle des Vorliegens von sauren oder basischen Gruppen in physiologisch verträgliche Salze umwandelt.

Die eingesetzten Ausgangsprodukte der Formel IV, Va und Vb und Reagenzien können entweder nach bekannten Verfahren hergestellt werden oder sind käuflich erhältlich.
Die Umsetzungen erfolgen beispielsweise durch Reaktion von 3,5 substituierten-1H-pyrazol-4-ylaminen (Verbindungen der Formel IV) mit Säuren (Verbindungen der Formel Va) nach der Carbodiimidmethode oder mit Säurechloriden (Verbindungen 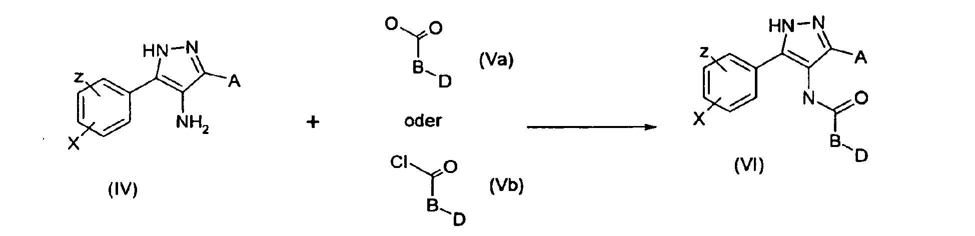 der Formel Vb) zu den entsprechenden Amiden (Verbindungen der Formel VI)

Aus Verbindungen der Formel VI können alle geschützten Verbindungen der Formel I in Anwesenheit von Phsphorpentoxid und Phosphoroxychlorid in einem Lösungsmittel, beispielsweise siedendem Xylol, hergestellt werden. Entschützung von funktionellen Gruppen und weitere Funtionalsierung erfolgt ausgehend von geschützten Verbindungen der Formel I.

Im Verfahrensschritt b) wird die Verbindung der Formel I sofern sie in diastereoisomerer oder enantiomerer Form auftritt und bei der gewählten Synthese als deren Gemische anfällt, in die reinen Stereoisomeren getrennt, entweder durch Chromatographie an einem gegebenenfalls chiralen Trägermaterial, oder, sofern die racemischen Verbindung der Formel 1 zur Salzbildung befähigt ist, durch fraktionierte Kristallisation der mit einer optisch aktiven Base oder Säure als Hilfsstoff gebildeten diastereomeren Salze. Als chirale Stationärphasen für die dünnschicht- oder säulenchromatographische Trennung von Enantiomeren eignen sich zum Beispiel modifizierte Kieselgelträger (sogenannte Pirkle-Phasen) sowie hochmolekulare Kohlenhydrate wie Triacetylcellulose. Für analytische Zwecke sind nach entsprechender, dem Fachmann bekannter Derivatisierung, auch gaschromatographische Methoden an chiralen Stationärphasen anwendbar. Zur Enantiomerentrennung der racemischen Carbonsäuren werden mit einer optisch aktiven, in der Regel kommerziell erhältlichen Base wie (-)-Nicotin, (+)- und (-)-Phenyl-ethylamin, Chininbasen, L-Lysin oder L-und D-Arginin die unterschiedlich löslichen diastereomeren Salze gebildet, die schwerer lösliche Komponente als Feststoff isoliert, das leichter lösliche Diastereomer aus der Mutterlauge abgeschieden, und aus den so gewonnenen diastereomeren Salzen die reinen Enantiomeren gewonnen. Auf prinzipiell gleiche Weise kann man die racemischen Verbindungen der Formel I, die eine basische Gruppe wie eine Aminogruppe enthalten, mit optisch aktiven Säuren, wie (+)-Campher-10-sulfonsäure, D- und L- Weinsäure, D-und L- Milchsäure sowie (+) und (-)-Mandelsäure in die reinen Enantiomeren überführen. Auch kann man chirale Verbindungen, die Alkohol- oder Amin-funktionen enthalten, mit entsprechend aktivierten oder gegebenenfalls N-geschützten enantiomerenreinen Aminosäuren in die entsprechenden Ester oder Amide, oder umgekehrt chirale Carbonsäuren mit carboxygeschützten enantiomerenreinen Aminosäuren in die Amide oder mit enantiomerenreinen Hydroxycarbonsäuren wie Milchsäure, in die entsprechenden chiralen Ester überführen. Sodann kann die Chiralität des in enantiomerenreiner Form eingebrachten Aminosäure- oder Alkoholrestes zur Trennung der Isomeren genutzt werden, indem man eine Trennung der nunmehr vorliegenden Diastereomeren durch Kristallisation oder Chromatographie an geeigneten Stationärphasen vornimmt und danach den mitgeführte chiralen Molekülteil mittels geeigneter Methoden wieder abspaltet.

Saure oder basische Produkte der Verbindung der Formel I können in Form ihrer Salze oder in freier Form vorliegen. Bevorzugt sind pharmakologisch verträgliche Salze, z. B. Alkali- oder Erdalkalimetallsalze bzw. Hydrochloride, Hydrobromide, Sulfate, Hemisulfate, alle möglichen Phosphate sowie Salze der Aminosäuren, natürlicher Basen oder Carbonsäuren.

Die Herstellung physiologisch verträglicher Salze aus zur Salzbildung befähigten Verbindungen der Formel I, einschließlich deren stereoisomeren Formen, gemäß Verfahrensschritt c) erfolgt in an sich bekannter Weise. Die Verbindungen der Formel I bilden mit basischen Reagenzien wie Hydroxiden, Carbonaten, Hydrogencarbonaten, Alkoholaten sowie Ammoniak oder organischen Basen, beispielsweise Trimethyl- oder Triethylamin, Ethanolamin oder Triethanolamin oder auch basischen Aminosäuren, etwa Lysin, Ornithin oder Arginin, stabile Alkali-, Erdalkali- oder gegebenenfalls substituierte Ammoniumsalze. Sofern die Verbindungen der Formel I basische Gruppen aufweisen, lassen sich mit starken Säuren auch stabile Säureadditionssalze herstellen. Hierfür kommen sowohl anorganische als auch organische Säuren wie Chlorwasserstoff-, Bromwasserstoff-, Schwefel-, Phosphor-, Methansulfon-, Benzolsulfon-, p-Toluolsulfon-, 4-Brombenzol-sulfon-, Cyclohexylamidosulfon-, Trifluormethylsulfon-, Essig-, Oxal-, Wein-, Bernstein- oder Trifluoressigsäure in Frage.

Die Erfindung betrifft auch Arzneimittel, gekennzeichnet durch einen wirksamen Gehalt an mindestens einer Verbindung der Formel I und/oder eines physiologisch verträglichen Salzes der Verbindung der Formel I und/oder eine gegebenenfalls stereoisomere Form der Verbindung der Formel I, zusammen mit einem pharmazeutisch geeigneten und physiologisch verträglichen Trägerstoff, Zusatzstoff und/oder anderen Wirk- und Hilfsstoffen.
Aufgrund der pharmakologischen Eigenschaften eignen sich die Verbindungen der Formel I zur Herstellung eines Arzneimittels zur Prophylaxe und Therapie all solcher Erkrankungen, an deren Verlauf eine verstärkte Aktivität von NFκB-induzierende Kinase beteiligt ist.

Die Erfindung betrifft ferner die Verwendung der Verbindung der Formel II 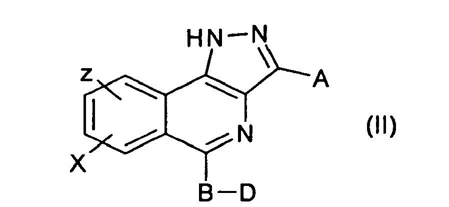 und/oder eine stereoisomere Form der Verbindung der Formel II und/oder ein physiologisch verträgliches Salz der Verbindung der Formel II, wobei
A für
   1. -(C₁-C₆)-Alkyl, worin Alkyl gerade oder verzweigt ist und ein- oder zweifach unabhängig voneinander substituiert ist durch
      1.1 -O-R1 oder
      1.2 C(O)-OR1, worin R1 für
         a) Wasserstoffatom oder
         b) -(C₁-C₆)-Alkyl steht,
   2. -O-R 1,
   3. C(O)-OR1,
   4. Heteroaryl mit 5 bis 14 Ringgliedern, worin Heteroaryl unsubstituiert oder substituiert ist,
   5. -(C₁-C₆)-Alkyl, worin Alkyl gerade oder verzweigt ist, oder
   6. -(C₆-C₁₄)-Aryl, worin Aryl unsubstituiert oder ein-, zwei- oder dreifach unabhängig voneinander substituiert ist durch R2, steht,
B für
   1. eine kovalente Bindung oder
   2. -(C₁-C₄)-Alkylen, worin Alkylen gerade oder verzweigt ist und ein- oder zweifach unabhängig voneinander durch R1 substituiert ist und R1 wie - oben definiert ist, steht,
D für
   1. Heteroaryl mit 5 bis 14 Ringgliedern, worin Heteroaryl unsubstituiert oder ein-, zwei- oder dreifach unabhängig voneinander substituiert ist durch R2, worin R2 für
      a) Wasserstoffatom,
      b) -(C₁-C₄)-Alkyl,
      c) -OH,
      d) -O-(C₁-C₄)-Alkyl,
      e) Halogen oder
      f) -N(R3)-R4, worin R3 und R4 unabhängig voneinander Wasserstoffatom oder -(C₁-C₄)-Alkyl bedeuten, steht,
   2. Heterocyclus mit 5 bis 12 Ringgliedern, worin Heterocyclus unsubstituiert oder ein-, zwei- oder dreifach unabhängig voneinander substituiert ist durch R2, und R2 wie oben definiert ist,
   3. -(C₆-C₁₄)-Aryl, worin Aryl unsubstituiert oder ein-, zwei- oder dreifach unabhängig voneinander substituiert ist durch R2, und R2 wie oben definiert ist, oder
   4. -(C₃-C₆)-Cycloalkyl, worin Cycloalkyl unsubstituiert oder ein-, zwei- oder dreifach unabhängig voneinander substituiert ist durch R2, und R2 wie oben definiert ist, steht, und
X und Z gleich oder verschieden sind und unabhängig voneinander für
   a) Wasserstoffatom,
   b) -(C₁-C₄)-Alkyl,
   c) -OH,
   d) -O-(C₁-C₄)-Alkyl oder
   e) Halogen stehen,
zur Herstellung eines Arzneimittels zur Prophylaxe und Therapie all solcher Erkrankungen, an deren Verlauf eine verstärkte Aktivität von NFκB-induzierende Kinase beteiligt ist.

Die Erfindung betrifft ferner die Verwendung der Verbindung der Formel II, wobei
A für
   1. -(C₁-C₃)-Alkyl, worin Alkyl gerade oder verzweigt ist und unsubstituiert ist oder ein- oder zweifach unabhängig voneinander substituiert ist durch
      1.1 -O-R1 oder
      1.2 C(O)-OR1 ,
         worin R1 für
         a) Wasserstoffatom oder
         b) -(C₁-C₃)-Alkyl steht,
   2. Phenyl oder
   3. C(O)-OR1, steht,
B für eine kovalente Bindung steht,
D für
   1. Phenyl, worin Phenyl unsubstituiert oder ein-, zwei- oder dreifach unabhängig voneinander substituiert ist durch R2, worin R2 für
      a) Wasserstoffatom,
      b) -(C₁-C₄)-Alkyl oder
      c) -N(R3)-R4, worin R3 und R4 unabhängig voneinander Wasserstoffatom oder -(C₁-C₃)-Alkyl bedeuten, steht,
   2. Pyridyl, worin Pyridyl unsubstituiert oder ein-, zwei- oder dreifach unabhängig voneinander substituiert ist durch R2 und R2 wie oben definiert ist, oder
   3. -(C₄-C₆)-Cycloalkyl, worin Cycloalkyl unsubstituiert oder ein-, zwei- oder dreifach unabhängig voneinander substituiert ist durch R2 und R2 wie oben definiert ist, steht, und
X und Z gleich oder verschieden sind und unabhängig voneinander für Wasserstoffatom oder Halogen stehen.

Zu den Erkrankungen, an deren Verlauf eine verstärkte Aktivität von NFκB-induzierende Kinase beteiligt ist, gehören beispielsweise degenerative Gelenkerkrankungen wie Osteoarthritis oder rheumatoide Arthritis. Weiterhin eignen sich die Verbindungen der Formel II zur Behandlung von Asthma und Transplantationen wie Abstoßungsreaktionen des Körpers gegen das übertragene Organ und auch Abstoßungsreaktionen des übertragenen Organs gegen den Körper, in welches das übertragene Organ verpflanzt wurde.

Die Applikation der erfindungsgemäßen Arzneimittel kann durch orale, inhalative, rektale oder transdermale Applikation oder durch subkutane, intraartikuläre, intraperitoneale oder intravenöse Injektion erfolgen. Bevorzugt ist die orale Applikation.

Die Erfindung betrifft auch ein Verfahren zur Herstellung eines Arzneimittels, das dadurch gekennzeichnet, dass man mindestens eine Verbindung der Formel 1 mit einem pharmazeutisch geeigneten und physiologisch verträglichen Träger und gegebenenfalls weiteren geeigneten Wirk-, Zusatz- oder Hilfsstoffen in eine geeignete Darreichungsform bringt.

Geeignete feste oder galenische Zubereitungsformen sind beispielsweise Granulate, Pulver, Dragees, Tabletten, (Mikro)Kapseln, Suppositorien, Sirupe, Säfte, Suspensionen, Emulsionen, Tropfen oder injizierbare Lösungen sowie Präparate mit protrahierter Wirkstoff-Freigabe, bei deren Herstellung übliche Hilfsmittel wie Trägerstoffe, Spreng-, Binde-, Überzugs-, Quellungs-, Gleit- oder Schmiermittel, Geschmacksstoffe, Süßungsmittel und Lösungsvermittler . Verwendung finden. Als häufig verwendete Hilfsstoffe seien Magnesiumcarbonat, Titandioxid, Laktose, Mannit und andere Zucker, Talkum, Milcheiweiß, Gelatine, Stärke, Cellulose und ihre Derivate, tierische und pflanzliche Öle wie Lebertran, Sonnenblumen-, Erdnuß- oder Sesamöl, Polyethylen-glykol und Lösungsmittel wie etwa steriles Wasser und ein- oder mehrwertige Alkohole wie Glycerin, genannt.

Vorzugsweise werden die pharmazeutischen Präparate in Dosierungseinheiten hergestellt und verabreicht, wobei jede Einheit als aktiven Bestandteil eine bestimmte Dosis der erfindungsgemäßen Verbindung der Formel I enthält. Bei festen Dosierungseinheiten wie Tabletten, Kapseln, Dragees oder Suppositorien, kann diese Dosis bis zu etwa 1000 mg, bevorzugt jedoch etwa 50 bis 300 mg und bei Injektionslösungen in Ampullenform bis zu etwa 300 mg, vorzugsweise aber etwa 10 bis 100 mg, betragen.

Für die Behandlung eines erwachsenen, etwa 70 kg schweren Patienten sind je nach Wirksamkeit der Verbindung gemäß Formel I, Tagesdosen von etwa 20 mg bis 1000 mg Wirkstoff, bevorzugt etwa 100 mg bis 500 mg indiziert. Unter Umständen können jedoch auch höhere oder niedrigere Tagesdosen angebracht sein. Die Verabreichung der Tagesdosis kann sowohl durch Einmalgabe in Form einer einzelnen Dosierungseinheit oder aber mehrerer kleinerer Dosierungseinheiten als auch durch Mehrfachgabe unterteilter Dosen in bestimmten Intervallen erfolgen.

Endprodukte werden in der Regel durch massenspektroskopische Methoden (FAB-, ESI-MS) bestimmt, angegeben sind jeweils der Hauptpeak. Temperaturangaben in Grad Celsius, RT bedeutet Raumtemperatur (22 °C bis 26 °C). Verwendete Abkürzungen sind entweder erläutert oder entsprechen den üblichen Konventionen.

Nachfolgend ist die Erfindung an Hand von Beispielen näher erläutert.

### Herstellungsbeispiele

### Beispiel 1 3,5-Diphenyl-1H-pyrazol[4,3-c]isoquinolin (1)

### a) N-(3,5-Diphenyl-1 H-pyrazol-4-yl)-benzylamin (2)

Zu einer Lösung von 260 mg Benzoesäure in 10 mL Methylenchlorid wurden 574 mg Hydroxybenzotriazol und 822 µL Diisopropylcarbodiimid gegeben, dazu wurde bei 0 °C 500 mg 3,5-Diphenyl-1H-pyrazol-4-ylamin in 2 mL Acetonitril zugetropft, 12 Stunden (h) bei Raumtemperatur (RT) gerührt, mit Wasser versetzt und mit Ethylacetat extrahiert. Die organische Phase wurde über Magnesiumsulfat getrocknet und unter verminderten Druck eingeengt. Der Rückstand enthielt die Titelverbindung und wurde in die Folgereaktion ohne weitere Aufreinigung eingesetzt.

### b) 3,5-Diphenyl-1H-pyrazol[4,3-c]isoquinolin (1)

Zu einer Lösung von 240 mg N-(3,5-Diphenyl-1H-pyrazol-4-yl)-benzylamin (2) in 10 mL Xylol wurden 201 mg Phosphorpentoxid gegeben, dazu wurden bei 150 °C 195 µl Phosphoroxychlorid zugetropft. Die Reaktionslösung wurde 4 h bei 150 °C gerührt, anschließend 12 h bei RT gerührt, mit gesättigter Natriumhydrogencarbonat-Lösung versetzt und mit Methylenchlorid extrahiert. Die organische Phase wurde über Magnesiumsulfat getrocknet, unter verminderten Druck eingeengt und über HPLC (Merk-Hibar-Lichrospher® 100-RP-18, Wasser (0,1 %Trifluoressigsäure)/ Acetonitril (0,1%Trifluoressigsäure) = 80/20 -> 10/90) gereinigt. Man erhielt:
1. C₂₂H₁₅N₃ (321,38) MS (ESI) 322 (M+H)

### Beispiel 2 5-(3-Methoxy-phenyl)-3-methyl-1H-pyrazolo[4,3-c]isoquinolin (3)

### a) 3-Methoxy-N-(3-methyl-5-phenyl-1 H-pyrazol-4-yl)-benzamid (4)

Zu einer Lösung von 160 mg 3-Methoxy-benzoesäure in 10 mL Dimethylformamid wurden 311 mg Hydroxybenzotriazol und 445 µL Diisopropylcarbodiimid gegeben, dazu wurden 200 mg 3-Methyl-5-phenyl-1H-pyrazol-4-ylamin zugegeben, 12 h bei RT gerührt, mit Wasser versetzt und mit Methylenchlorid extrahiert. Die organische Phase wurde über Magnesiumsulfat getrocknet, unter verminderten Druck eingeengt und über HPLC (Merk-Hibar-Lichrospher 100-RP-18, Wasser (0,1%Trifluoressigsäure)/ Acetonitril (0,1%Trifluoressigsäure) = 80/20 -> 10/90) gereinigt. Man erhielt: 4 C₁₇H₁₅N₃O₂ (293,33) MS (ESI) 294 (M+H)

### b) 5-(3-Methoxy-phenyl)-3-methyl-1 H-pyrazolo[4,3-c]isoquinolin (3)

128 mg 3-Methoxy-N-(3-methyl-5-phenyl-1 H-pyrazol-4-yl)-benzamid (4) wurden in 5 mL Xylol suspendiert, bei 160 °C gekocht, dazu wurde 119 mg Phosphorpentoxid gegeben, und 15 min bei 160 °C nachgerüht. Zu der Suspension wurden 27 µL Phosphoroxychlorid zugetropft, 12 h bei 160°C gerührt, mit gesättigter Natriumhydrogencarbonat-Lösung versetzt und mit Ethylacetat extrahiert. Die organische Phase wurde über Magnesiumsulfat getrocknet, unter verminderten Druck eingeengt und über HPLC (Merk-Hibar-Lichrospher® 100-RP-18, Wasser (0,1%Trifluoressigsäure)/ Acetonitril (0,1%Trifluoressigsäure) = 80/20 -> 10/90) gereinigt. Man erhielt: 3. C₁₈H₁₅N₃O (298,34) MS (ESI) 290 (M+H)

### Beispiel 3 3-(3-Methyl-1H-pyrazolo[4,3-c]isoquinolin-5-yl)-phenol (5)

Zu einer Lösung von 55 mg 5-(3-Methoxy-phenyl)-3-methyl-1H-pyrazolo[4,3-c]isoquinoline (3) in 3 mL Methylenchlorid wurden bei -78 °C 380 µL 1 M Bortribromid-Lösung in Methylenchlorid zugetropft. Die Reaktionslösung wurde 12 h bei RT gerührt, unter verminderten Druck eingeengt und über HPLC (Merk-Hibar-Lichrospher® 100-RP-18, Wasser (0,1%Trifluoressigsäure)/ Acetonitril (0,1%Trifluoressigsäure) = 80/20 -> 10/90) gereinigt. Man erhielt:
5. C₁₇H₁₃N₃O (275,31) MS (ESI) 276 (M+H)

### Beispiel 4 5-(2-Methoxy-phenyl)-3-methyl-1H-pyrazol[4,3-c]isoquinolin (6)

### a) 2-Methoxy-N-(3-methyl-5-phenyl-1H-pyrazol-4-yl)-benzamid (7)

Die Herstellung erfolgte analog zu Beispiel 2 a), ausgehend von 175 mg 2-Methoxybenzoesäure. Man erhielt: 7. C₁₈H₁₇N₃O₂ (307,36) MS (ESI) 308 (M+H)

### b) 5-(2-Methoxy-phenyl)-3-methyl-1H-pyrazol[4,3-c]isoquinolin (6)

Die Herstellung erfolgte analog zu Beispiel 1 b), ausgehend von 90 mg 2-Methoxy-N-(3-methyl-5-phenyl-1H-pyrazol-4-yl)-benzamid (7). Man erhielt: 6.
C₁₈H₁₅N₃O (289,34) MS (ESI) 290 (M+H)

### Beispiel 5 5-(2,3-Dimethoxy-phenyl)-3-methyl-1H-pyrazol[4,3-c]isoquinolin (8)

### a) 2,3-Dimethoxy-N-(3-methyl-5-phenyl-1H-pyrazol-4-yl)-benzamide (9)

Die Herstellung erfolgte analog zu Beispiel 2 a), ausgehend von 210 mg 2,3-Dimethoxybenzoesäure. Man erhielt: 9. C₁₉H₁₉N₃ O₃ (337,38) MS (ESI) 338 (M+H)

### b) 5-(2,3-Dimethoxy-phenyl)-3-methyl-1H-pyrazol[4,3-c]isoquinolin (8)

Die Herstellung erfolgte analog zu Beispiel 1 b), ausgehend von 40 mg 2,3-Dimethoxy-N-(3-methyl-5-phenyl-1H-pyrazol-4-yl)-benzamide (9). Man erhielt: 8.
C₁₉H₁₇N₃O₂ (319,37) MS (ESI) 320 (M+H)

### Beispiel 6 5-(2,4-Dimethoxy-phenyl)-3-methyl-1H-pyrazol[4,3-c]isoquinolin (10)

### a) 2,4-Dimethoxy-N-(3-methyl-5-phenyl-1H-pyrazol-4-yl)-benzamid (11)

Die Herstellung erfolgte analog zu Beispiel 2 a), ausgehend von 210 mg 2,4-Dimethoxybenzoesäure. Man erhielt: 11. C₁₉H₁₉N₃O₃ (337,38) MS (ESI) 338 (M+H)

### b) 5-(2,4-Dimethoxy-phenyl)-3-methyl-1H-pyrazol[4,3-c]isoquinolin (10)

Die Herstellung erfolgte analog zu Beispiel 1 b), ausgehend von 87 mg 2,4-Dimethoxy-N-(3-methyl-5-phenyl-1H-pyrazol-4-yl)-benzamide (11). Man erhielt: 10.
C₁₉H₁₇N₃O₂ (319,37) MS (ESI) 320 (M+H)

### Beispiel 7 5-(2,6-Dimethoxy-phenyl)-3-methyl-1H-pyrazol[4,3-c]isoquinolin (12)

### a) 2,6-Dimethoxy-N-(3-methyl-5-phenyl-1 H-pyrazol-4-yl)-benzamid (13)

Die Herstellung erfolgte analog zu Beispiel 2 a), ausgehend von 210 mg 2,6-Dimethoxybenzoesäure. Man erhielt: 13. C₁₉H₁₉N₃O₃ (337,38) MS (ESI) 338 (M+H)

### b) 5-(2,6-Dimethoxy-phenyl)-3-methyl-1 H-pyrazol[4,3-c]isoquinolin (12)

Die Herstellung erfolgte analog zu Beispiel 1 b), ausgehend von 100 mg 2,6-Dimethoxy-N-(3-methyl-5-phenyl-1H-pyrazol-4-yl)-benzamid (13). Man erhielt: 12
C₁₉H₁₇N₃O₂ (319,37) MS (ESI) 320 (M+H)

### Beispiel 8 5-(3,4-Dimethoxy-phenyl)-3-methyl-1H-pyrazol[4,3-c]isoquinolin (14)

### a) 3,4-Dimethoxy-N-(3-methyl-5-phenyl-1 H-pyrazol-4-yl)-benzamid (15)

Die Herstellung erfolgte analog zu Beispiel 2 a), ausgehend von 210 mg 3,4-Dimethoxybenzoesäure. Man erhielt: 15. C₁₉H₁₉N₃ O₃ (337,38) MS (ESI) 338 (M+H)

### b) 5-(3,4-Dimethoxy-phenyl)-3-methyl-1H-pyrazol[4,3-c]isoquinolin (14)

Die Herstellung erfolgte analog zu Beispiel 1 b), ausgehend von 100 mg 3,4-Dimethoxy-N-(3-methyl-5-phenyl-1 H-pyrazol-4-yl)-benzamid (15). Man erhielt: 14.
C₁₉H₁₇N₃O₂ (319,37) MS (ESI) 320 (M+H)

### Beispiel 9 5-(3,5-Dimethoxy-phenyl)-3-methyl-1 H-pyrazol[4,3-c]isoquinolin (16)

### a) 3,5-Dimethoxy-N-(3-methyl-5-phenyl-1H-pyrazol-4-yl)-benzamid (17)

Die Herstellung erfolgte analog zu Beispiel 2 a), ausgehend von 210 mg 3,5-Dimethoxybenzoesäure. Man erhielt: 17. C₁₉H₁₉N₃ O₃ (337,38) MS (ESI) 338 (M+H)

### b) 5-(3,5-Dimethoxy-phenyl)-3-methyl-1H-pyrazol[4,3-c]isoquinolin (16)

Die Herstellung erfolgte analog zu Beispiel 1 b), ausgehend von 60 mg 3,5-Dimethoxy-N-(3-methyl-5-phenyl-1 H-pyrazol-4-yl)-benzamid (17). Man erhielt: 16.
C₁₉H₁₇N₃O₂ (319,37) MS (ESI) 320 (M+H)

### Beispiel 10 5-(2,3,4-Trimethoxy-phenyl)-3-methyl-1H-pyrazol[4,3-c]isoquinolin (18)

### a) 2,3,4-Trimethoxy-N-(3-methyl-5-phenyl-1H-pyrazol-4-yl)-benzamid (19)

Die Herstellung erfolgte analog zu Beispiel 2 a), ausgehend von 244 mg 2,3,4-Trimethoxybenzoesäure. Man erhielt: 19. C₂₀H₂₁N₃O₄ (367,41) MS (ESI) 368 (M+H)

### b) 5-(2,3,4-Trimethoxy-phenyl)-3-methyl-1H-pyrazol[4,3-c]isoquinolin (18)

Die Herstellung erfolgte analog zu Beispiel 1 b), ausgehend von 73 mg 2,3,4-Trimethoxy-N-(3-methyl-5-phenyl-1 H-pyrazol-4-yl)-benzamid (19). Man erhielt: 18
C₂₀H₁₉N₃O₃ (349,39) MS (ESI) 350 (M+H)

### Beispiel 11 5-(2,4,6-Trimethoxy-phenyl)-3-methyl-1H-pyrazol[4,3-c]isoquinolin (20)

### a) 2,4,6-Trimethoxy-N-(3-methyl-5-phenyl-1 H-pyrazol-4-yl)-benzamid (21)

Die Herstellung erfolgte analog zu Beispiel 2 a), ausgehend von 244 mg 2,4,6-Trimethoxybenzoesäure. Man erhielt: 21. C₂₀H₂₁N₃O₄ (367,41) MS (ESI) 368 (M+H)

### b) 5-(2,4,6-Trimethoxy-phenyl)-3-methyl-1 H-pyrazol[4,3-c]isoquinolin (18)

Die Herstellung erfolgte analog zu Beispiel 1 b) , ausgehend von 63 mg 2,4,6-Trimethoxy-N-(3-methyl-5-phenyl-1 H-pyrazol-4-yl)-benzamid (21). Man erhielt: 20
C₂₀H₁₉N₃O₃ (349,39) MS (ESI) 350 (M+H)

### Beispiel 12 5-(3,4,5-Trimethoxy-phenyl)-3-methyl-1H-pyrazol[4,3-c]isoquinolin (22)

### a) 3,4,5-Trimethoxy-N-(3-methyl-5-phenyl-1 H-pyrazol-4-yl)-benzamid (23)

Die Herstellung erfolgte analog zu Beispiel 2 a), ausgehend von 244 mg 3,4,5-Trimethoxybenzoesäure. Man erhielt: 23. C₂₀H₂₁N₃O₄ (367,41) MS (ESI) 368 (M+H)

### b) 5-(3,4,5-Trimethoxy-phenyl)-3-methyl-1H-pyrazol[4,3-c]isoquinolin (22)

Die Herstellung erfolgte analog zu Beispiel 1 b) , ausgehend von 65 mg 3,4,5-Trimethoxy-N-(3-methyl-5-phenyl-1 H-pyrazol-4-yl)-benzamid (23) Man erhielt: 22 C₂₀H₁₉N₃O₃ (349,39) MS (ESI) 350 (M+H)

### Beispiel 13 5-(2-Ethoxy-phenyl)-3-methyl-1H-pyrazol[4,3-c]isoquinolin (24)

### a) 2-Ethoxy-N-(3-methyl-5-phenyl-1H-pyrazol-4-yl)-benzamid (25)

Die Herstellung erfolgte analog zu Beispiel 2 a), ausgehend von 191 mg 2-Ethoxybenzoesäure. Man erhielt: 25. C₁₉H₁₉N₃O₂ (321,38) MS (ESI) 322 (M+H)

### b) 5-(2-Ethoxy-phenyl)-3-methyl-1H-pyrazol[4,3-c]isoquinolin (24)

Die Herstellung erfolgte analog zu Beispiel 1 b) , ausgehend von 60 mg 2-Ethoxy-N-(3-methyl-5-phenyl-1 H-pyrazol-4-yl)-benzamid (25). Man erhielt: 24
C₁₉H₁₇N₃O (303,37) MS (ESI) 304 (M+H)

### Beispiel 14 5-(4-Diethylamino-phenyl)-3-methyl-1H-pyrazol[4,3-c]isoquinolin (26)

### a) 4-Diethylamino-N-(3-methyl-5-phenyl-1H-pyrazol-4-yl)-benzamid (27)

Die Herstellung erfolgte analog zu Beispiel 2 a), ausgehend von 222 mg 4-Diethylaminobenzoesäure. Man erhielt: 27. C₂₁H₂₄N₄O (348,45) MS (ESI) 349 (M+H)

### b) 5-(4-Diethylamino-phenyl)-3-methyl-1H-pyrazol[4,3-c]isoquinolin (26)

Die Herstellung erfolgte analog zu Beispiel 1 b), ausgehend von 130 mg 4-Diethylamino-N-(3-methyl-5-phenyl-1H-pyrazol-4-yl)-benzamid (27). Man erhielt: 26
C₂₁ H₂₂N₄ (330,44) MS (ESI) 331 (M+H)

### Beispiel 15 3-Methyl-5-pyridin-4-yl-1H-pyrazol[4,3-c]isoquinolin (28)

### a) N-(3-Methyl-5-phenyl-1H-pyrazol-4-yl)-isonicotinamid (29)

Zu einer Lösung von 200 mg 3-Methyl-5-phenyl-1 H-pyrazol-4-ylamin in 2 mL Pyridin wurden 205 mg lsonicotinsäurechlorid*HCl zugefügt, anschließend wurde 12 h bei RT gerührt, mit Wasser versetzt und mit Methylenchlorid extrahiert. Die organische Phase wurde über Magnesiumsulfat getrocknet, unter verminderten Druck eingeengt und über HPLC (Merk-Hibar-Lichrospher® 100-RP-18, Wasser (0,1%Trifluoressigsäure)/ Acetonitril (0,1%Trifluoressigsäure) = 80/20 -> 10/90) gereinigt. Man erhielt: 27 C₁₆H₁₄N₄O (278,32) MS (ESI) 279 (M+H)

### b) 3-Methyl-5-pyridin-4-yl-1H-pyrazol[4,3-c]isoquinolin (28)

Die Herstellung erfolgte analog zu Beispiel 1 b), ausgehend von 110 mg N-(3-Methyl-5-phenyl-1 H-pyrazol-4-yl)-isonicotinamid (29). Man erhielt: 28
C₁₆H₁₂N₄ (260,30) MS (ESI) 261 (M+H)

### Beispiel 16 3-Methyl-5-pyridin-3-yl-1H-pyrazol[4,3-c]isoquinolin (30)

### a) N-(3-Methyl-5-phenyl-1H-pyrazol-4-yl)-nicotinamid (31)

Die Herstellung erfolgte analog zu Beispiel 15 a), ausgehend von 205 mg Nicotinsäurechlorid*HCl. Man erhielt: 31. C₁₆H₁₄N₄O (278,32) MS (ESI) 279 (M+H)

### b) 3-Methyl-5-pyridin-3-yl-1H-pyrazol[4,3-c]isoquinolin (30)

Die Herstellung erfolgte analog zu Beispiel 1 b), ausgehend von 140 mg N-(3-Methyl-5-phenyl-1 H-pyrazol-4-yl)-nicotinamid (31). Man erhielt: 30
C₁₆H₁₂N₄ (260,30) MS (ESI) 261 (M+H)

### Beispiel 17 3-Methyl-5-pyridin-2-yl-1H-pyrazol[4,3-c]isoquinolin (32)

### a) Pyridine-2-carboxylic acid (3-methyl-5-phenyl-1H-pyrazol-4-yl)-amid (33)

Die Herstellung erfolgte analog zu Beispiel 15 a), ausgehend von 205 mg Pyridin-2-carbonyl chlorid*HCl. Man erhielt: 33 C₁₆H₁₄N₄O (278,32) MS (ESI) 279 (M+H)

### b) 3-Methyl-5-pyridin-2-yl-1H-pyrazol[4,3-c]isoquinolin (32)

Die Herstellung erfolgte analog zu Beispiel 1 b), ausgehend von 110 mg Pyridin-2-carboxylic acid (3-methyl-5-phenyl-1H-pyrazol-4-yl)-amid (33). Man erhielt: 32
C₁₆H₁₂N₄ (260,30) MS (ESI) 261 (M+H)

### Beispiel 18 5-Benzyl-3-methyl-1H-pyrazol[4,3-c]isoquinolin (34)

### a) N-(3-Methyl-5-phenyl-1H-pyrazol-4-yl)-2-phenyl-acetamid (35)

Die Herstellung erfolgte analog zu Beispiel 15 a), ausgehend von 152 µL Phenylacetylchlorid. Man erhielt: 35. C₁₈H₁₇N₃O (291,36) MS (ESI) 292 (M+H)

### b) 5-Benzyl-3-methyl-1H-pyrazol[4,3-c]isoquinolin (34)

Die Herstellung erfolgte analog zu Beispiel 1 b), mit 50 mg N-(3-Methyl-5-phenyl-1 H-pyrazol-4-yl)-2-phenyl-acetamid (35). Man erhielt: 34 C₁₈H₁₅N₃ (273,34)MS (ESI) 274 (M+H)

### Beispiel 19 3-Methyl-5-phenethyl-1H-pyrazol[4,3-c]isoquinolin (36)

### a) N-(3-Methyl-5-phenyl-1 H-pyrazol-4-yl)-3-phenyl-propionamid (37)

Die Herstellung erfolgte analog zu Beispiel 15 a), ausgehend von 127 µL 3-Phenylpropionsäurechlorid. Man erhielt: 37. C₁₉H₁₉N₃O (305,38) MS (ESI) 306 (M+H)

### b) 3-Methyl-5-phenethyl-1H-pyrazol[4,3-c]isoquinolin (36)

Die Herstellung erfolgte analog zu Beispiel 1 b), ausgehend von 110 mg N-(3-Methyl-5-phenyl-1H-pyrazol-4-yl)-3-phenyl-propionamid (37). Man erhielt: 36
C₁₈H₁₅N₃ (287,37) MS (ESI) 288 (M+H)

### Beispiel 20 3-Methyl-5-(1-methyl-piperidin-4-yl)-1H-pyrazol[4,3-c]isoquinolin (38)

a) 1-Methyl-piperidine-4-carboxylic acid (3-methyl-5-phenyl-1H-pyrazol-4-yl)-amid (39) Die Herstellung erfolgte analog zu Beispiel 2 a), ausgehend von 207 mg 1-Methylpiperidin-4-carbonsäure*HCl und 197 µL Diisopropylethylamin. Man erhielt: 39
C₁₇H₂₂N₄O (298,39) MS (ESI) 299 (M+H)

### b) 3-Methyl-5-(1-methyl-piperidin-4-yl)-1H-pyrazol[4,3-c]isoquinolin (38)

Die Herstellung erfolgte analog zu Beispiel 1 b) , ausgehend von 220 mg 1-Methyl-piperidin-4-carboxylsäure (3-methyl-5-phenyl-1H-pyrazol-4-yl)-amid (39). Man erhielt: 38
C₁₇H₂₀N₄ (280,38) MS (ESI) 281 (M+H)

### Beispiel 21 7,8-Dimethoxy-3-methyl-5-phenyl-1H-pyrazol[4,3-c]isoquinolin (40)

### a) 1-(3,4-Dimethoxy-phenyl)-butan-1,3-dion (41)

3,9 g Natriumhydrid wurden in 150 mL Cyclohexan vorgelegt, dazu wurde eine Lösung aus 15 g 1-(3,4-Dimethoxy-phenyl)-ethanon in 16,3 mL Ethylacetat zugegeben. Die Reaktionslösung wurde 1 h bei 80 °C gekocht, anschließend mit Essigsäure versetzt und mit MTB-Ether extrahiert. Die MTB-Ether Phase wurde über Magnesiumsulfat getrocknet und einrotiert. Rückstand wurde über Kieselgel (Ethylacetat/Heptan 1/6) chromatographiert. Man erhielt: 41 C₁₂H₁₄O₄ (222,24) MS (ESI) 223 (M+H)

### b) 1-(3,4-Dimethoxy-phenyl)-butan-1,2,3-trion 2-oxim (42)

5 g 1-(3,4-Dimethoxy-phenyl)-butane-1,3-dione (41) wurden in 25 mL Essigsäure vorgelegt, bei 15°C wurden 1,71 g Natriumnitrit in 5 mL Wasser gelöst zugetropft. Man rührte 1 h bei RT, anschließend blieb die Reaktionslösung 2 h stehen. Zu der Lösung wurden 50 g Eis zugegeben, dann wurde das ganze 12 h bei 0 °C gelagert, wobei das Produkt ausfiel. Anschließend wurde abgesaugt und bei 50°C im Trockenschrank getrocknet. Man erhielt: 42
C₁₂H₁₃NO₅ (251,24) MS (ESI) 252 (M+H)

### a) 5-(3,4-Dimethoxy-phenyl)-3-methyl-4-nitro-1H-pyrazol (43)

6,77 g 1-(3,4-Dimethoxy-phenyl)-butane-1,2,3-trione 2-oxime (42) wurden in 54 mL Essigsäure gelöst, bei RT wurden 0,96 g Hydrazin zugetropft und 2 h bei 60°C nachgerührt. In die Reaktionslösung wurde Eis gegeben, mit Natriumcarbonat neutralisiert und mit MTB-Ether extrahiert. Die organische Phase wurde über Magnesiumsulfat getrocknet und einrotiert. Man erhielt: 43. C₁₂H₁₃N₃O₃ (247,26) MS (ESI) 248 (M+H)

### a) 5-(3,4-Dimethoxy-phenyl)-3-methyl-1H-pyrazol-4-ylamin (44)

4,84 g 5-(3,4-Dimethoxy-phenyl)-3-methyl-4-nitro-1H-pyrazol (43) wurden in 80 mL Ethanol gelöst und 0,5 g Pd/C wurden dazugegeben. Die Lösung wurde 2 h unter Wasserstoff geschüttelt, anschließend über Magnesiumsulfat filtriert und einrotiert. Der Rückstand wurde über Kieselgel (Ethylacetat/Heptan 3/1) chromatographiert. Man erhielt: 44
C₁₂H₁₅N₃O₂ (233,27) MS (ESI) 234 (M+H)

### e) N-[5-(3,4-Dimethoxy-phenyl)-3-methyl-1 H-pyrazol-4-yl]-benzamid (45)

Die Herstellung erfolgte analog zu Beispiel 15 a), ausgehend von 500 mg 5-(3,4-Dimethoxyphenyl)-3-methyl-1H-pyrazol-4-ylamine (44) und 280 µL Benzoylchlorid. Man erhielt: 45
C₁₉H₁₉N₃O₃ (337,38) MS (ESI) 338 (M+H)

### a) 7,8-Dimethoxy-3-methyl-5-phenyl-1H-pyrazol[4,3-c]isoquinolin (40)

Die Herstellung erfolgte analog zu Beispiel 1 b), ausgehend von 187 mg N-[5-(3,4-Dimethoxyphenyl)-3-methyl-1 H-pyrazol-4-yl]-benzamid (45). Man erhielt: 40
C₁₉H₁₇N₃O₂ (319,37) MS (ESI) 320 (M+H)

### Beispiel 22 7-Methoxy-3-methyl-5-phenyl-1H-pyrazol[4,3-c]isoquinolin (46)

### a) 1-(4-Methoxy-phenyl)-butan-1,3-dion (47)

Die Herstellung erfolgte analog zu Beispiel 21 a), ausgehend von 15 g 1-(4-Methoxy-phenyl)-ethanon. Man erhielt: 47. C₁₁H₁₂O₃ (192,22) MS (ESI) 193 (M+H)

### b) 1-(4-Methoxy-phenyl)-butan-1,2,3-trione 2-oxim (48)

Die Herstellung erfolgte analog zu Beispiel 21 b), ausgehend von 5 g 1-(4-Methoxy-phenyl)-butan-1,3-dion (47). Man erhielt: 48
C₁₁H₁₁NO₄ (221,21) MS (ESI) 222 (M+H)

### a) 5-(4-Methoxy-phenyl)-3-methyl-4-nitroso-1H-pyrazole (49)

Die Herstellung erfolgte analog zu Beispiel 21 c), ausgehend von 4,19 g 1-(4-Methoxy-phenyl)-butan-1,2,3-trione 2-oxime (48).
Man erhielt: 49. C₁₁H₁₁N₃O₂ (217,23) MS (ESI) 218 (M+H)

### a) 5-(4-Methoxy-phenyl)-3-methyl-1 H-pyrazol-4-ylamin (50)

Die Herstellung erfolgte analog zu Beispiel 21 d), ausgehend von 3,17 g 5-(4-Methoxy-phenyl)-3-methyl-4-nitro-1H-pyrazol (49). Man erhielt: 50. C₁₁H₁₃N₃O (203,25) MS (ESI) 204 (M+H)

### e) N-[5-(4-Methoxy-phenyl)-3-methyl-1 H-pyrazol-4-yl]-benzamid (51)

Die Herstellung erfolgte analog zu Beispiel 15 a), ausgehend von 500 mg 5-(4-Methoxy-phenyl)-3-methyl-1H-pyrazol-4-ylamin (50) und 315 µl Benzoylchlorid. Man erhielt: 51
C₁₈H17N₃O₂ (307,36) MS (ESI) 308 (M+H)

### a) 7-Methoxy-3-methyl-5-phenyl-1H-pyrazol[4,3-c]isoquinolin (46)

Die Herstellung erfolgte analog zu Beispiel 1 b), ausgehend von 230 mg N-[5-(4-Methoxyphenyl)-3-methyl-1 H-pyrazol-4-yl]-benzamd (51). Man erhielt: 46
C₁₈H₁₅N₃O (289,34) MS (ESI) 290 (M+H)

### Beispiel 23 7,8-Dimethoxy-5-(3-methoxy-phenyl)-3-methyl-1 H-pyrazol[4,3-c]isoquinolin (52)

a) N-[5-(3,4-Dimethoxy-phenyl)-3-methyl-1H-pyrazol-4-yl]-3-methoxy-benzamid (53) Die Herstellung erfolgt analog zu Beispiel 15 a), ausgehend von 300 mg 5-(3,4-Dimethoxyphenyl)-3-methyl-1 H-pyrazol-4-ylamin (44) und 242 mg 3-Methoxy-benzoesäurechlorid. Man erhielt: 53. C₂₀H₂₁N₃O₄ (367,41) MS (ESI) 368 (M+H)

### b) 7,8-Dimethoxy-5-(3-methoxy-phenyl)-3-methyl-1 H-pyrazol[4,3-c]isoquinolin (52)

Die Herstellung erfolgte analog zu Beispiel 1 b), ausgehend von 291 mg N-[5-(3,4-Dimethoxyphenyl)-3-methyl-1 H-pyrazol-4-yl]-3-methoxy-benzamid (53). Man erhielt: 52
C₂₀H₁₉N₃O₃ (349,39) MS (ESI) 350 (M+H)

### Beispiel 24 7,8-Dimethoxy-3-methyl-5-pyridin-2-yl-1H-pyrazol[4,3-c]isoquinolin (54)

### a) Pyrid ine-2-carboxylic acid [5-(3,4-dimethoxy-phenyl)-3-methyl-1H-pyrazol-4-yl]-amid (55)

Die Herstellung erfolgte analog zu Beispiel 15 a), ausgehend von 200 mg 5-(3,4-Dimethoxyphenyl)-3-methyl-1H-pyrazol-4-ylamin (44) und 168 mg Pyridin-2-carbonylchlorid * HCl. Man erhielt: 55. C₁₈H₁₈N₄O₃ (338,37) MS (ESI) 339 (M+H)

### b) 7,8-Dimethoxy-3-methyl-5-pyridin-2-yl-1H-pyrazol[4,3-c]isoquinolin (54)

Die Herstellung erfolgte analog zu Beispiel 1 b), ausgehend von 160 mg Pyridine-2-carboxylic acid [5-(3,4-dimethoxy-phenyl)-3-methyl-1 H-pyrazol-4-yl]- amid (55). Man erhielt: 54
C₂₀H₁₉N₃O₃ (320,35) MS (ESI) 321 (M+H)

### Beispiel 25 7,8-Dimethoxy-3-methyl-5-pyridin-3-yl-1H-pyrazol[4,3-c]isoquinolin (56)

### a) N-[5-(3,4-Dimethoxy-phenyl)-3-methyl-1H-pyrazol-4-yl]-nicotinamid (57)

Die Herstellung erfolgte analog zu Beispiel 15 a), ausgehend von 200 mg 5-(3,4-Dimethoxyphenyl)-3-methyl-1H-pyrazol-4-ylamine (44) und 168 mg Nicotinsäurechlorid*HCl. Man erhielt: 57. C₁₈H₁₈N₄O₃ (338,37) MS (ESI) 339 (M+H)

### b) 7,8-Dimethoxy-3-methyl-5-pyridin-3-yl-1H-pyrazol[4,3-c]isoquinolin (56)

Die Herstellung erfolgte analog zu Beispiel 1 b), ausgehend von 126 mg N-[5-(3,4-Dimethoxyphenyl)-3-methyl-1 H-pyrazol-4-yl]-nicotinamid (57). Man erhielt: 56 C₂₀H₁₉N₃O₃ (320,35) MS (ESI) 321 (M+H)

### Beispiel 26 7-Methoxy-5-(3-methoxy-phenyl)-3-methyl-1H-pyrazol[4,3-c]isoquinolin (58)

### a) 3-Methoxy-N-[5-(4-methoxy-phenyl)-3-methyl-1H-pyrazol-4-yl]-benzamid (59)

Die Herstellung erfolgte analog zu Beispiel 15 a), ausgehend von 300 mg 5-(4-Methoxy-phenyl)-3-methyl-1 H-pyrazol-4-ylamin (50) und 277 mg 3-Methoxy-benzoesäurechlorid. Man erhielt: 59. C₁₉H₁₉N₃O₃ (337,38) MS (ESI) 338 (M+H)

### b) 7-Methoxy-5-(3-methoxy-phenyl)-3-methyl-1 H-pyrazol[4,3-c]isoquinolin (58)

Die Herstellung erfolgte analog zu Beispiel 1 b), ausgehend von 263 mg 3-Methoxy-N-[5-(4-methoxy-phenyl)-3-methyl-1 H-pyrazol-4-yl]-benzamid (59). Man erhielt: 58 C₁₉H₁₇N₃O₂ (319,37) MS (ESI) 320 (M+H)

### Beispiel 27 5-Phenyl-1H-pyrazol[4,3-c]isoquinoline-3-carbonsäure (60)

Zu einer Lösung von 600 mg 5-phenyl-3-methyl-1 H-pyrazol[4,3-c]isoquinolin in 36 mL Pyridin wurden 2,1 g Kaliumpermanganat in 36 mL Wasser zugegeben. Der Ansatz wurde bei 40 °C 12 h gerührt. Die entstandene Suspension wurde über Kieselgel abgesaugt, unter verminderten Druck eingeengt und über HPLC (Merk-Hibar-Lichrospher® 100-RP-18, Wasser (0,1%Trifluoressigsäure)/ Acetonitril (0,1%Trifluoressigsäure) = 80/20 -> 10/90) gereinigt. Man erhielt: 60. C₁₇H₁₁ N₃O₂ (289,30) MS (ESI) 290 (M+H)

### Beispiel 28 5-Phenyl-1H-pyrazol[4,3-c]isoquinoline-3-carbonsäuremethylester (61)

18 µL Thionylchlorid wurden in 0,5 mL Methanol vorgelegt und 30 min gerührt. Dazu wurden 19 mg 5-Phenyl-1H-pyrazol[4,3-c]isoquinolin-3-carbonsäure (60) in 0,5 mL Methanol zugetropft. Der Ansatz wurde 12 h bei RT gerührt, mit gesättigter Natriumhydrogencarbonat-Lösung versetzt und jeweils 1x mit Ethylacetat und Methylenchlorid extrahiert. Die organischen Phasen wurden vereinigt über Magnesiumsulfat getrocknet, unter verminderten Druck eingeengt und über HPLC (Merck-Hibar-Lichrospher 100-RP-18, Wasser (0,1%Trifluoressigsäure)/ Acetonitril (0,1%Trifluoressigsäure) = 80/20 -> 10/90) gereinigt. Man erhielt: 61. C₁₈H₁₃N₃O₂ (303,32) MS (ESI) 304 (M+H)

### Beispie 29 (5-Phenyl-1H-pyrazol[4,3-c]isoquinolin-3-yl)-methanol (62)

7,5 mg Lithiumaluminiumhydrid wurden in 1,5 mL Tetrahydrofuran vorgelegt und 10 min gerührt. Dazu wurden 6 mg 5-Phenyl-1 H-pyrazol[4,3-c]isoquinolin-3-carbonsäuremethylester (61) in 1,5 mL Tetrahydrofuran zugetropft. Der Ansatz wurde 3 h bei 80 °C gerührt, mit Wasser versetzt und mit Methylenchlorid extrahiert. Die organische Phase wurde über Magnesiumsulfat getrocknet, unter verminderten Druck eingeengt und über HPLC (Merck-Hibar-Lichrospher® 100-RP-18, Wasser (0,1%Trifluoressigsäure)/ Acetonitril (0,1%Trifluoressigsäure) = 80/20 -> 10/90) gereinigt. Man erhielt: 62
C₁₇H₁₃N₃O₁ (275,31) MS (ESI) 276 (M+H)

### Beispiel 30 2-(3-Methyl-1H-pyrazol[4,3-c]isoquinolin-5-yl)-phenol (63)

Die Herstellung erfolgt analog zu Beispiel 3, mit 210 mg 5-(2-Methoxy-phenyl)-3-methyl-1H-pyrazol[4,3-c]isoquinolin (6). Man erhielt: 63. C₁₇H₁₃N₃O (275,31) MS (ESI) 276 (M+H)

### Beispiel 31 4-(3-Methyl-1H-pyrazol[4,3-c]isoquinolin-5-yl)-benzen-2,4-diol (64)

Die Herstellung erfolgte analog zu Beispiel 3, ausgehend von 9 mg 5-(3,5-Dimethoxy-phenyl)-3-methyl-1H-pyrazol[4,3-c]isoquinolin (16). Man erhielt: 64
C₁₇H₁₃N₃O₂ (291,31) MS (ESI) 292 (M+H)

### Beispiel 32 4-(3-Methyl-1H-pyrazol[4,3-c]isoquinolin-5-yl)-benzen-1,2-diol (65)

Die Herstellung erfolgte analog zu Beispiel 3, ausgehend von 40 mg 5-(2,3-Dimethoxy-phenyl)-3-methyl-1H-pyrazol[4,3-c]isoquinolin (8). Man erhielt: 65
C₁₇H₁₃N₃O₂ (291,31) MS (ESI) 292 (M+H)

### Pharmakologische Beispiele

Die erfindungsgemäßen Pyrazoloisoquinolinderivate wurden in verschiedenen in vitro Assay Systemen auf inhibitorische Aktivität gegen NIK getestet. Dabei wurden humane periphere Blutlymphocyten für 1 h mit unterschiedlichen Konzentrationen der Verbindungen vorinkubiert und dann für 24 h mit LPS oder IL1β stimuliert. Danach wurde im Kulturüberstand die Freisetzung von TNFα mittels eines kommerziell erhältlichen ELISA Test Kits gemessen und die IC50 für die jeweilige Verbindung bestimmt. Die Zytotoxizität wurde über LDH Freisetzung mittels eines kommerziell erhältlichen Test Kits gemessen und die LD50 für die jeweilige Verbindung bestimmt. In einem weiteren Ansatz wurde humanes heparinisiertes Vollblut für 1 h mit unterschied-lichen Konzentrationen der Verbindungen vorinkubiert und dann für 24 h mit LPS stimuliert. Nach 24 h wurde im Überstand die Freisetzung von IL1β, TNFα und IL6 mit kommerziell erhältlichen Test Kits gemessen und die 1C50 für die jeweilige Verbindung bestimmt.

Die nachfolgenden Tabellen 1, 2 und 3 zeigen die Ergebnisse.

**Tabelle 1: Inhibition der TNFα Freisetzung in LPS-stimulierten humanen peripheren Blutlymphocyten:**

| Beispiel Nr. | TNFα Freisetzung IC50 (µM) | IL6 Freisetzung IC50 (µM) | Zytotoxizität LD50 (µM) |
|---|---|---|---|
| 1 | 1,9 | 80 | >100 |
| 2 | 9 | >100 | >100 |
| 3 | 7,5 | 80 | >100 |

| | | | |
|---|---|---|---|
| Das Zeichen ">" bedeutet größer als | | | |

**Tabelle 2: Inhibition der TNFα Freisetzung in IL1β-stimulierten humanen peripheren Blutlymphocyten:**

| Beispiel Nr. | TNFα Freisetzung IC50 (µM) | IL6 Freisetzung IC50 (µM) | Zytotoxizität LD50 (µM) |
|---|---|---|---|
| 1 | 5 | 80 | >100 |
| 2 | 35 | >100 | >100 |
| 3 | 8 | >100 | >100 |

**Tabelle 3: Inhibition der IL1 β, TNFα und IL6 Freisetzung in LPS-stimuliertem heparinisiertem humanem Vollblut:**

| Beispiel Nr. | IL1β Freisetzung IC50 (µM) | TNFα Freisetzung IC50 (µM) | IL6 Freisetzung IC50 (µM) |
|---|---|---|---|
| 1 | 33 | 12.5 | >100 |
| 2 | 1.3 | 1.2 | 7 |
| 3 | 29 | 5 | >100 |

## Patentansprüche

1. Verbindung der Formel I 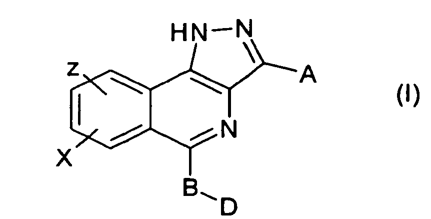 und/oder eine stereoisomere Form der Verbindung der Formel I und/oder ein physiologisch verträgliches Salz der Verbindung der Formel I, wobei
A für
1. -(C₁-C₆)-Alkyl, worin Alkyl gerade oder verzweigt ist und ein- oder zweifach unabhängig voneinander substituiert ist durch
1.1 -O-R1 oder
1.2 C(O)-OR1, worin R1 für
a) Wasserstoffatom oder
b) -(C₁-C₆)-Alkyl steht,
2. -O-R1,
3. C(O)-OR1,
4. Heteroaryl mit 5 bis 14 Ringgliedern, worin Heteroaryl unsubstituiert oder ein-, zwei- oder dreifach unabhängig voneinander durch R2 substituiert ist, steht, oder
5. -(C₁-C₆)-Alkyl, worin Alkyl gerade oder verzweigt ist,
B für
1. eine kovalente Bindung oder
2. -(C₁-C₄)-Alkylen, worin Alkylen gerade oder verzweigt ist und ein- oder
zweifach unabhängig voneinander durch R1 substituiert ist und R1 wie oben definiert ist, steht,
D für
1. Heteroaryl mit 5 bis 14 Ringgliedern, worin Heteroaryl unsubstituiert
oder ein-, zwei- oder dreifach unabhängig voneinander substituiert ist durch R2,
worin R2 für
a) Wasserstoffatom,
b) -(C₁-C₄)-Alkyl,
c) -OH,
d) -O-(C₁-C₄)-Alkyl,
e) Halogen oder
f) -N(R3)-R4, worin R3 und R4 unabhängig voneinander Wasserstoffatom oder -(C₁-C₄)-Alkyl bedeuten, steht,
2. Heterocyclus mit 5 bis 12 Ringgliedern, worin Heterocyclus unsubstituiert oder ein-, zwei- oder dreifach unabhängig voneinander substituiert ist durch R2, und R2 wie oben definiert ist,
3. -(C₆-C₁₄)-Aryl, worin Aryl ein-, zwei- oder dreifach unabhängig voneinander substituiert ist durch
a) -OH,
b) -O-(C₁-C₄)-Alkyl, oder
c) -N(R3)-R4, worin R3 und R4 unabhängig voneinander Wasserstoffatom oder -(C₁-C₄)-Alkyl bedeuten, oder
4. -(C₃-C₆)-Cycloalkyl, worin Cycloalkyl unsubstituiert oder ein-, zwei- oder dreifach unabhängig voneinander substituiert ist durch R2, und R2 wie oben definiert ist, steht, und
X und Z gleich oder verschieden sind und unabhängig voneinander für
a) Wasserstoffatom,
b) -(C₁-C₄)-Alkyl,
c) -OH,
d) -O-(C₁-C₄)-Alkyl oder
e) Halogen stehen.

2. Verbindung der Formel I gemäß Anspruch 1, wobei
A für
1. -(C₁-C₃)-Alkyl, worin Alkyl gerade oder verzweigt ist und ein- oder zweifach unabhängig voneinander substituiert ist durch
1.1 -O-R1 oder
1.2 -C(O)-OR1, worin R1 für
a) Wasserstoffatom oder
b) -(C₁-C₃)-Alkyl steht, oder
2. C(O)-OR1, steht,
B für eine kovalente Bindung steht,
D für
1. Phenyl, worin Phenyl unsubstituiert oder ein-, zwei- oder dreifach unabhängig voneinander substituiert ist durch R2,
worin R2 für
a) Wasserstoffatom,
b) -(C₁-C₄)-Alkyl oder
c) -N(R3)-R4, worin R3 und R4 unabhängig voneinander Wasserstoffatom oder -(C₁-C₃)-Alkyl bedeuten, steht,
2. Pyridyl, worin Pyridyl unsubstituiert oder ein-, zwei- oder dreifach unabhängig voneinander substituiert ist durch R2 und R2 wie oben definiert ist, oder
3. -(C₄-C₆)-Cycloalkyl, worin Cycloalkyl unsubstituiert oder ein-, zwei- oder dreifach unabhängig voneinander substituiert ist durch R2 und R2 wie oben definiert ist, steht, und
X und Z gleich oder verschieden sind und unabhängig voneinander für Wasserstoffatom oder Halogen stehen.

3. Verbindung der Formel I gemäß Anspruch 1, **dadurch gekennzeichnet, dass** die Verbindung der Formel I aus der Gruppe
5-(3-Methoxy-phenyl)-3-methyl-1H-pyrazolo[4,3-c]isoquinolin,
3-(3-Methyl-1H-pyrazolo[4,3-c]isoquinolin-5-yl)-phenol,
5-(2-Methoxy-phenyl)-3-methyl-1H-pyrazol[4,3-c]isoquinolin,
5-(2,3-Dimethoxy-phenyl)-3-methyl-1 H-pyrazol[4,3-c]isoquinolin,
5-(2,4-Dimethoxy-phenyl)-3-methyl-1 H-pyrazol[4,3-c]isoquinolin,
5-(2,6-Dimethoxy-phenyl)-3-methyl-1H-pyrazol[4,3-c]isoquinolin,
5-(3,4-Dimethoxy-phenyl)-3-methyl-1H-pyrazol[4,3-c]isoquinolin,
5-(3,5-Dimethoxy-phenyl)-3-methyl-1H-pyrazol[4,3-c]isoquinolin,
5-(2,3,4-Trimethoxy-phenyl)-3-methyl-1H-pyrazol[4,3-c]isoquinolin,
5-(2,4,6-Trimethoxy-phenyl)-3-methyl-1H-pyrazol[4,3-c]isoquinolin,
5-(3,4,5-Trimethoxy-phenyl)-3-methyl-1H-pyrazol[4,3-c]isoquinolin,
5-(2-Ethoxy-phenyl)-3-methyl-1H-pyrazol[4,3-c]isoquinolin,
5-(4-Diethylamino-phenyl)-3-methyl-1 H-pyrazol[4,3-c]isoquinolin,
3-Methyl-5-pyridin-4-yl-1 H-pyrazol[4,3-c]isoquinolin,
3-Methyl-5-pyridin-3-yl-1 H-pyrazol[4,3-c]isoquinolin,
3-Methyl-5-pyridin-2-yl-1 H-pyrazol[4,3-c]isoquinolin,
3-Methyl-5-(1-methyl-piperidin-4-yl)-1 H-pyrazol[4,3-c]isoquinolin,
7,8-Dimethoxy-5-(3-methoxy-phenyl)-3-methyl-1 H-pyrazol[4,3-c]isoquinolin,
7,8-Dimethoxy-3-methyl-5-pyridin-2-yl-1 H-pyrazol[4,3-c]isoquinolin,
7,8-Dimethoxy-3-methyl-5-pyridin-3-yl-1 H-pyrazol[4,3-c]isoquinolin,
7-Methoxy-5-(3-methoxy-phenyl)-3-methyl-1 H-pyrazol[4,3-c]isoquinolin,
2-(3-Methyl-1 H-pyrazol[4,3-c]isoquinolin-5-yl)-phenol,
4-(3-Methyl-1 H-pyrazol[4,3-c]isoquinolin-5-yl)-benzen-2,4-diol oder
4-(3-Methyl-1 H-pyrazol[4,3-c]isoquinolin-5-yl)-benzen-1,2-diol ausgewählt ist.

4. Verfahren zur Herstellung der Verbindung der Formel I gemäß einem oder mehreren der Ansprüche 1 bis 3, **dadurch gekennzeichnet, dass** man
a) eine Verbindung der Formel IV 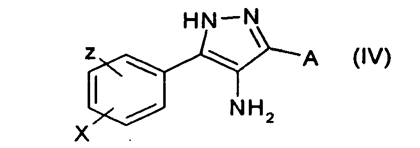 mit einer Verbindung der Formeln Va oder Vb 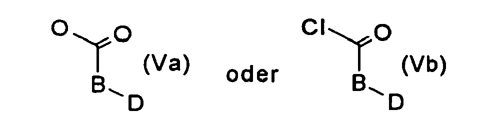 zu einer Verbindung der Formel VI umsetzt 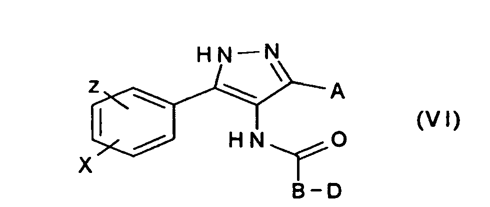 und in Anwesenheit von Phsphorpentoxid und Phosphoroxychlorid in eine geschützte Verbindung der Formel I umsetzt, und abschließend die Schutzgruppe abspaltet, wobei X, Z, A, B und D die in Anspruch 1 genannte Bedeutung haben,
b) eine nach Verfahren a) hergestellte Verbindung der Formel 1, die aufgrund ihrer chemischen Struktur in enantiomeren Formen auftritt, durch Salzbildung mit enantiomerenreinen Säuren oder Basen, Chromatographie an chiralen Stationärphasen oder Derivatisierung mittels chiraler enantiomerenreinen Verbindungen wie Aminosäuren, Trennung der somit erhaltenen Diastereomeren, und Abspaltung der chiralen Hilfsgruppen in die reinen Enantiomeren auftrennt, oder
c) die nach den Verfahren a) oder b) hergestellte Verbindung der Formel 1 entweder in freier Form isoliert oder im Falle des Vorliegens von sauren oder basischen Gruppen in physiologisch verträgliche Salze
umwandelt.

5. Arzneimittel, **gekennzeichnet durch** einen wirksamen Gehalt an mindestens einer Verbindung der Formel I gemäß einem oder mehreren der Ansprüche 1 bis 3 zusammen mit einem pharmazeutisch geeigneten und physiologisch verträglichen Trägerstoff, Zusatzstoff und/oder anderen Wirk- und Hilfsstoffen.

6. Verwendung der Verbindung der Formel II 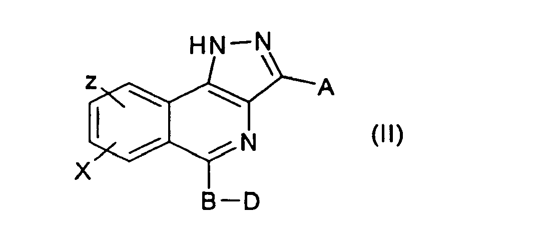 und/oder eine stereoisomere Form der Verbindung der Formel II und/oder ein physiologisch verträgliches Salz der Verbindung der Formel II, wobei
A für
1. -(C₁-C₆)-Alkyl, worin Alkyl gerade oder verzweigt ist und ein- oder zweifach unabhängig voneinander substituiert ist durch
1.1 -O-R1 oder
1.2 C(O)-OR1 , worin R1 für
a) Wasserstoffatom oder
b) -(C₁-C₆)-Alkyl steht,
2. -O-R1,
3. C(O)-OR1,
4. Heteroaryl mit 5 bis 14 Ringgliedern, worin Heteroaryl unsubstituiert oder substituiert ist,
5. -(C₁-C₆)-Alkyl, worin Alkyl gerade oder verzweigt ist, oder
6. -(C₆-C₁₄)-Aryl, worin Aryl unsubstituiert oder ein-, zwei- oder dreifach unabhängig voneinander substituiert ist durch R2, steht,
B
für 1. eine kovalente Bindung oder
2. -(C₁-C₄)-Alkylen, worin Alkylen gerade oder verzweigt ist und ein- oder zweifach unabhängig voneinander durch R1 substituiert ist und R1 wie oben definiert ist, steht,
D für
1. Heteroaryl mit 5 bis 14 Ringgliedern, worin Heteroaryl unsubstituiert oder ein-, zwei- oder dreifach unabhängig voneinander substituiert durch R2 ist, worin R2 für
a) Wasserstoffatom,
b) -(C₁-C₄)-Alkyl,
c) -OH,
d) -O-(C₁-C₄)-Alkyl,
e) Halogen oder
f) -N(R3)-R4, worin R3 und R4 unabhängig voneinander Wasserstoffatom oder-(C₁-C₄)-Alkyl bedeuten, steht,
2. Heterocyclus mit 5 bis 12 Ringgliedern, worin Heterocyclus unsubstituiert oder ein-, zwei- oder dreifach unabhängig voneinander substituiert ist durch R2, und R2 wie oben definiert ist,
3. -(C₆-C₁₄)-Aryl, worin Aryl unsubstituiert oder ein-, zwei- oder dreifach unabhängig voneinander substituiert ist durch R2, und R2 wie oben definiert ist, oder
4. -(C₃-C₆)-Cycloalkyl, worin Cycloalkyl unsubstituiert oder ein-, zwei- oder dreifach unabhängig voneinander substituiert ist durch R2, und R2 wie oben definiert ist, steht, und
X und Z gleich oder verschieden sind und unabhängig voneinander für
1. Wasserstoffatom,
2. -(C₁-C₄)-Alkyl,
3. -OH,
4. -O-(C₁-C₄)-Alkyl oder
5. Halogen stehen,
zur Herstellung eines Arzneimittels zur Prophylaxe und Therapie von Osteoarthritis, oder Abstoßungsreaktionen des Körpers gegen ein übertragenes Organ oder Abstoßungsreaktionen des übertragenen Organs gegen den Körper.

7. Verwendung der Verbindung der Formel II gemäß Anspruch 6, wobei
A für
1. -(C₁-C₃)-Alkyl, worin Alkyl gerade oder verzweigt ist und unsubstituiert ist oder ein- oder zweifach unabhängig voneinander substituiert ist durch
1.1 -O-R1 oder
1.2 C(O)-OR1 ,
worin R1 für
a) Wasserstoffatom oder
b) -(C₁-C₃)-Alkyl steht,
2. Phenyl oder
3. C(O)-OR1, steht,
B für eine kovalente Bindung steht,
D für
1. Phenyl, worin Phenyl unsubstituiert oder ein-, zwei- oder dreifach unabhängig voneinander durch R2 substituiert ist, worin R2 für
a) Wasserstoffatom,
b) -(C₁-C₄)-Alkyl oder
c) -N(R3)-R4, worin R3 und R4 unabhängig voneinander Wasserstoffatom oder -(C₁-C₃)-Alkyl bedeuten, steht,
2. Pyridyl, worin Pyridyl unsubstituiert oder ein-, zwei- oder dreifach unabhängig voneinander substituiert ist durch R2 und R2 wie oben definiert ist, oder
3. -(C₄-C₆)-Cycloalkyl, worin Cycloalkyl unsubstituiert oder ein-, zwei- oder dreifach unabhängig voneinander substituiert ist durch R2 und R2 wie oben definiert ist, steht, und
X und Z gleich oder verschieden sind und unabhängig voneinander für Wasserstoffatom oder Halogen stehen.

## Claims

1. A compound of the formula I 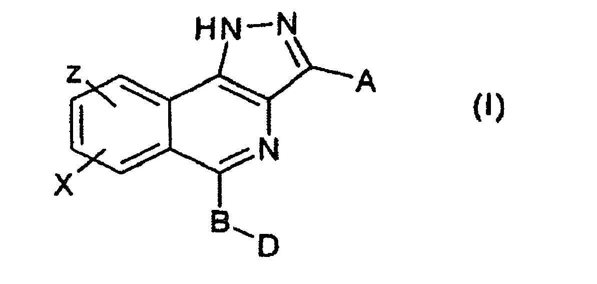 and/or a stereoisomeric form of the compound of the formula I and/or a physiologically tolerated salt of the compound of the formula I, wherein
A is
1. -(C₁-C₆)-alkyl, in which alkyl is straight-chain or branched and is substituted, once or twice, independently of each other, by
1.1 -O-R1 or
1.2 C(O)-OR1, in which R1 is
a) hydrogen atom or
b) -(C₁-C₆)-alkyl,
2. -O-R 1,
3. C(O)-OR1, or
4. heteroaryl having from 5 to 14 ring members, in which heteroaryl is unsubstituted or substituted, once, twice or three times, independently of each other, by R2, or
5. -(C₁-C₆)-alkyl, in which alkyl is straight-chain or branched,
B is
1. a covalent bond, or
2. -(C₁-C₄)-alkylene, in which alkylene is straight-chain or branched and is substituted, once or twice, independently of each other, by R1, and R1 is defined as above,
D is
1. heteroaryl having from 5 to 14 ring members, in which heteroaryl is unsubstituted or is substituted once, twice or three times, independently of each other, by R2,
in which R2 is
a) hydrogen atom,
b) -(C₁-C₄)-alkyl,
c) -OH,
d) -O-(C₁-C₄)-alkyl,
e) halogen, or
f) -N(R3)-R4, in which R3 and R4 are, independently of each other, hydrogen atom or -(C₁-C₄)-alkyl,
2. heterocycle having from 5 to 12 ring members, in which heterocycle is unsubstituted or substituted, once, twice or three times, independently of each other, by R2, and R2 is defined as above,
3. -(C₆-C₁₄)-aryl, in which aryl is substituted, once, twice or three times, independently of each other, by
a) -OH,
b) -O-(C₁-C₄)-alkyl, or
c) -N(R3)-R4, in which R3 and R4 are, independently of each other, hydrogen atom or -(C₁-C₄)-alkyl, or
4. -(C₃-C₆)-cycloalkyl, in which cycloalkyl is unsubstituted or substituted, once, twice or three times, independently of each other, by R2, and R2 is defined as above, and
X and Z are identical or different and are, independently of each other,
a) hydrogen atom,
b) -(C₁-C₄)-alkyl,
c) -OH,
d) -O-(C₁-C₄)-alkyl, or
e) halogen.

2. A compound of the formula I as claimed in claim 1, wherein
A is
1. -(C₁-C₃)-alkyl, in which alkyl is straight-chain or branched and is substituted, once or twice, independently of each other, by
1.1 -O-R1, or
1.2 -C(O)-OR1, in which R1 is
a) hydrogen atom, or
b) -(C₁-C₃)-alkyl, or
2. C(O)-OR1,
B is a covalent bond,
D is
1. phenyl, in which phenyl is unsubstituted or substituted, once, twice or three times, independently of each other, by R2,
in which R2 is
a) hydrogen atom,
b) -(C₁-C₄)-alkyl or
c) -N(R3)-R4, in which R3 and R4 are, independently of each other, hydrogen atom or -(C₁-C₃)-alkyl,
2. pyridyl, in which pryidyl is unsubstituted or substituted, once, twice or three times, independently of each other, by R2, and R2 is defined as above, or
3. -(C₄-C₆)-cycloalkyl, in which cycloalkyl is unsubstituted or substituted, once, twice or three times, independently of each other, by R2, and R2 is defined as above, and
X and Z are identical or different and are, independently of each other, hydrogen atom or halogen.

3. A compound of the formula 1 as claimed in claim 1, wherein the compound of the formula I is selected from the group
5-(3-methoxyphenyl)-3-methyl-1H-pyrazolo[4,3-c]isoquinoline,
3-(3-methyl-1 H-pyrazolo[4,3-c]isoquinolin-5-yl)phenol,
5-(2-methoxyphenyl)-3-methyl-1 H-pyrazolo[4,3-c]isoquinoline,
5-(2,3-dimethoxyphenyl)-3-methyl-1 H-pyrazolo[4,3-c]isoquinoline,
5-(2,4-dimethoxyphenyl)-3-methyl-1 H-pyrazolo[4,3-c]isoquinoline,
5-(2,6-dimethoxyphenyl)-3-methyl-1 H-pyrazolo[4,3-c]isoquinoline,
5-(3,4-dimethoxyphenyl)-3-methyl-1 H-pyrazolo[4,3-c]isoquinoline,
5-(3,5-dimethoxyphenyl)-3-methyl-1H-pyrazolo[4,3-c]isoquinoline,
5-(2,3,4-trimethoxyphenyl)-3-methyl-1 H-pyrazolo[4,3-c]isoquinoline,
5-(2,4,6-trimethoxyphenyl)-3-methyl-1 H-pyrazolo[4,3-c]isoquinoline,
5-(3,4,5-trimethoxyphenyl)-3-methyl-1H-pyrazolo[4,3-c]isoquinoline,
5-(2-ethoxyphenyl)-3-methyl-1 H-pyrazolo[4,3-c]isoquinoline,
5-(4-diethylaminophenyl)-3-methyl-1 H-pyrazolo[4,3-c]isoquinoline,
3-methyl-5-pyridin-4-yl-1H-pyrazolo[4,3-c]isoquinoline,
3-methyl-5-pyridin-3-yl-1H-pyrazolo[4,3-c]isoquinoline,
3-methyl-5-pyridin-2-yl-1H-pyrazolo[4,3-c]isoquinoline,
3-methyl-5-(1-methylpiperidin-4-yl)-1H-pyrazolo[4,3-c]isoquinoline,
7,8-dimethoxy-5-(3-methoxyphenyl)-3-methyl-1H-pyrazolo[4,3-c]isoquinoline,
7,8-dimethoxy-3-methyl-5-pyridin-2-yl-1H-pyrazolo[4,3-c]isoquinoline,
7,8-dimethxoy-3-methyl-5-pyridin-3-yl-1H-pyrazolo[4,3-c]isoquinoline,
7-methoxy-5-(3-methoxyphenyl)-3-methyl-1H-pyrazolo[4,3-c]isoquinoline,
2-(3-methyl-1H-pyrazolo[4,3-c]isoquinolin-5-yl)phenol,
4-(3-methyl-1 H-pyrazolo[4,3-c]isoquinolin-5-yl)benzene-2,4-diol, or
4-(3-methyl-1 H-pyrazolo[4,3-c]isoquinolin-5-yl)benzene-1,2-diol.

4. A process for preparing a compound of the formula I as claimed in one or more of claims 1 to 3, which comprises
a) reacting a compound of the formula IV 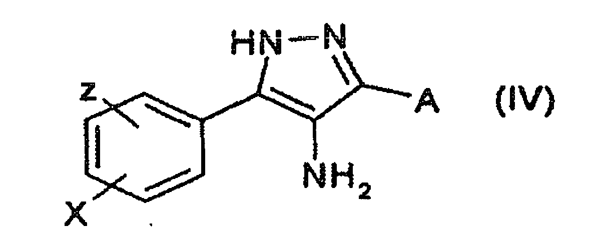 with a compound of the formula Va or Vb 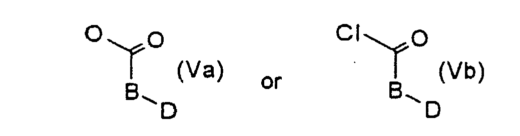 to give a compound of the formula VI 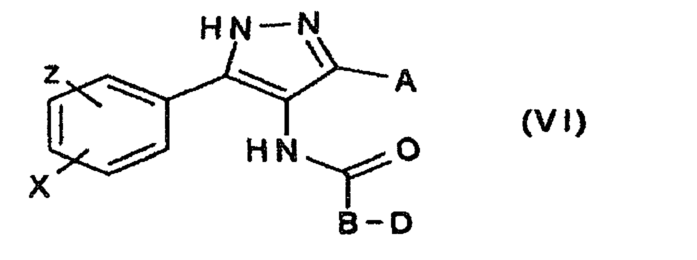 and, in the presence of phosphorus pentoxide and phosphoroxy chloride, converting the latter into a protected compound of the formula I, and, in conclusion, eliminating the protecting group, where X, Z, A, B and D are as defined in claim 1,
b) resolving a compound of the formula I, which has been prepared in accordance with method a) and which, on account of its chemical structure, appears in enantiomeric forms, into the pure enantiomers by means of salt formation with enantiomerically pure acids or bases, chromatography on chiral stationary phases or derivatization using chiral enantiomerically pure compounds, such as amino acids, separating the resulting diastereomers and eliminating the chiral auxiliary groups, or
c) either isolating the compound of the formula I, which has been prepared in accordance with methods a) or b), in free form or, when acidic or basic groups are present, converting it into physiologically tolerated salts.

5. A pharmaceutical which has an effective content of at least one compound of the formula I as claimed in one or more of claims 1 to 3 together with a pharmaceutically suitable and physiologically tolerated carrier substance, additive and/or other active compounds and auxiliary substances.

6. The use of the compound of the formula II 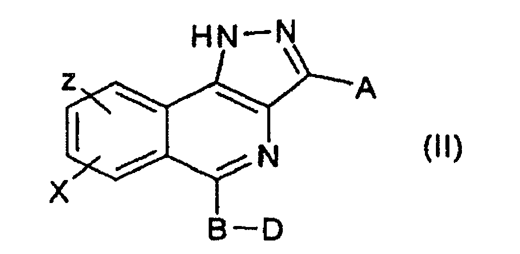 and/or a stereoisomeric form of the compound of formula II and/or a physiologically tolerated salt of the compound of the formula II, where
A is
1. -(C₁-C₆)-alkyl in which alkyl is straight-chain or branched and substituted once or twice, independently of each other, by
1.1 -O-R1 or
1.2 C(O)-OR1, in which R1 is
a) hydrogen atom or
b) -(C₁-C₆)-alkyl,
2. -O-R1,
3. C(O)-OR1,
4. heteroaryl having from 5 to 14 ring members, in which heteroaryl is unsubstituted or substituted,
5. -(C₁-C₆)-alkyl, in which alkyl is straight-chain or branched, or
6. -(C₆-C₁₄)-aryl, in which aryl is unsubstituted or substituted once, twice or three times, independently of each other, by R2,
B is
1. a covalent bond, or
2. -(C₁-C₄)-alkylene, in which alkylene is straight-chain or branched and substituted once or twice, independently of each other, by R1, and R1 is defined as above,
D is
1. heteroaryl having from 5 to 14 ring members, in which heteroaryl is unsubstituted or substituted once, twice or three times, independently of each other, by R2, in which R2 is
a) hydrogen atom,
b) -(C₁-C₄)-alkyl,
c) -OH,
d) -O-(C₁-C₄)-alkyl,
e) halogen, or
f) -N(R3)-R4, in which R3 and R4 are, independently of each other, hydrogen atom or -(C₁-C₄)-alkyl,
2. heterocycle having from 5 to 12 ring members, in which heterocycle is unsubstituted or substituted, once, twice or three times, independently of each other, by R2, and R2 is defined as above,
3. -(C₆-C₁₄)-aryl, in which aryl is unsubstituted or substituted once, twice or three times, independently of each other, by R2, and R2 is defined as above, or
4. -(C₃-C₆)-cycloalkyl, in which cycloalkyl is unsubstituted or substituted once, twice or three times, independently of each other, by R2, and R2 is defined as above, and
X and Z are identical or different and are, independently of each other,
1. hydrogen atom,
2. -(C₁-C₄)-alkyl,
3. -OH,
4. -O-(C₁-C₄)-alkyl, or
5. halogen,
for producing a pharmaceutical for the prophylaxis and therapy of osteoarthritis, or rejection reactions on the part of the body against a transplanted organ or rejection reactions on the part of the transplanted organ against the body.

7. The use of the compound formula II as claimed in claim 6, wherein
A is
1. -(C₁-C₃)-alkyl, in which alkyl is straight-chain or branched and is unsubstituted or substituted, once or twice, independently of each other, by
1.1 -O-R1, or
1.2. C(O)-OR1,
in which R1 is
a) hydrogen atom, or
b) -(C₁-C₃)-alkyl,
2. phenyl, or
3. C(O)-OR1,
B is a covalent bond,
D is
1. phenyl, in which phenyl is unsubstituted or substituted once, twice or three times, independently of each other, by R2, in which R2 is
a) hydrogen atom,
b) -(C₁-C₄)-alkyl, or
c) -N(R3)-R4, in which R3 and R4 are, independently of each other, hydrogen atom or -(C₁-C₃)-alkyl,
2. pyridyl, in which pyridyl is unsubstituted or substituted once, twice or three times, independently of each other, by R2 and R2 is defined as above, or
3. -(C₄-C₆)-cycloalkyl, in which cycloalkyl is unsubstituted or substituted once, twice or three times, independently of each other, by R2 and R2 is defined as above, and
X and Z are identical or different and are, independently of each other, hydrogen atom or halogen.

## Revendications

1. Composé de formule I 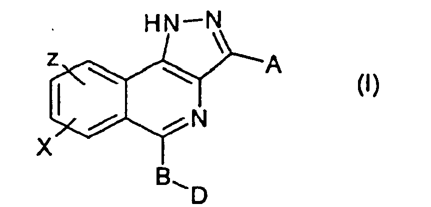 et/ou une forme stéréo-isomère du composé de formule I et/ou un sel physiologiquement acceptable du composé de formule I, où
A représente
1. -(C₁-C₆)-alkyle, où alkyle est linéaire ou ramifié et est monosubstitué ou disubstitué, indépendamment l'un de l'autre, par
1.1 -O-R1 ou
1.2 C(O)-OR1, où R1 représente
a) un atome d'hydrogène ou
b) - (C₁-C₆)-alkyle,
2. -O-R1
3. C(O)-OR1,
4. hétéroaryle comprenant 5 à 14 chaînons de cycle, où hétéroaryle est non substitué ou monosubstitué, disubstitué ou trisubstitué, indépendamment l'un de l'autre, par R2, ou
5. -(C₁-C₆)-alkyle, où alkyle est linéaire ou ramifié,
B représente
1. une liaison covalente ou
2. -(C₁-C₄)-alkylène, où alkylène est linéaire ou ramifié et est monosubstitué ou disubstitué, indépendamment l'un de l'autre, par R1 et R1 est défini comme ci-dessus,
D représente
1. hétéroaryle comprenant 5 à 14 chaînons de cycle, où hétéroaryle est non substitué ou monosubstitué, disubstitué ou trisubstitué, indépendamment l'un de l'autre, par R2
où R2 représente
a) un atome d'hydrogène
b) - (C₁-C₄)-alkyle,
c) -OH,
d) -O-(C₁-C₄)-alkyle,
e) halogène ou
f) -N(R3)-R4, où R3 et R4 signifient, indépendamment l'un de l'autre, un atome d'hydrogène ou - (C₁-C₄)-alkyle,
2. hétérocycle comprenant 5 à 12 chaînons de cycle, où hétérocycle est non substitué ou monosubstitué, disubstitué ou trisubstitué, indépendamment l'un de l'autre, par R2 et R2 est défini comme ci-dessus,
3. -(C₆-C₁₄)-aryle, où aryle est monosubstitué, disubstitué ou trisubstitué, indépendamment l'un de l'autre par
a) -OH,
b) -O-(C₁-C₄)-alkyle, ou
c) -N(R3)-R4, où R3 et R4 signifient, indépendamment l'un de l'autre, un atome d'hydrogène ou - -(C₁-C₄)-alkyle, ou
4. -(C₃-C₆)-cycloalkyle, où cycloalkyle est non substitué ou monosubstitué, disubstitué ou trisubstitué par R2, et R2 est défini comme ci-dessus, et
X et Z sont identiques ou différents et représentent, indépendamment l'un de l'autre,
a) un atome d'hydrogène
b) - (C₁-C₄)-alkyle,
c) -OH,
d) -O-(C₁-C₄)-alkyle ou
e) halogène.

2. Composé de formule I selon la revendication 1, où
A représente
1. -(C₁-C₃)-alkyle, où alkyle est linéaire ou ramifié et est monosubstitué ou disubstitué, indépendamment l'un de l'autre, par
1.1 -O-R1 ou
1.2 -C(O)-OR1, où R1 représente
a) un atome d'hydrogène ou
b) - (C₁-C₃)-alkyle, ou
2. C(O)-OR1,
B représente une liaison covalente
D représente
1. phényle, où phényle est non substitué ou monosubstitué, disubstitué ou trisubstitué, indépendamment l'un de l'autre, par R2, où R2 représente
a) un atome d'hydrogène
b) -(C₁-C₄)-alkyle ou
c) -N(R3)-R4, où R3 et R4 signifient, indépendamment l'un de l'autre, un atome d'hydrogène ou -(C₁-C₃)-alkyle, ou
2. pyridyle, où pyridyle est non substitué ou monosubstitué, disubstitué ou trisubstitué, indépendamment l'un de l'autre, par R2, et R2 est défini comme ci-dessus, ou
3. -(C₄-C₆)-cycloalkyle, où cycloalkyle est non substitué ou monosubstitué, disubstitué ou trisubstitué, indépendamment l'un de l'autre, par R2, et R2 est défini comme ci-dessus, et
X et Z sont identiques ou différents et représentent, indépendamment l'un de l'autre, un atome d'hydrogène ou halogène.

3. Composé de formule I selon la revendication 1, **caractérisé en ce que** le composé de formule I est choisi dans le groupe formé par la 5-(3-méthoxyphényl)-3-méthyl-1H-pyrazolo[4,3-c]isoquinoléine, le 3-(3-méthyl-1H-pyrazolo[4,3-c]isoquinoléin-5-yl)-phénol, la 5-(2-méthoxyphényl)-3-méthyl-1H-pyrazol[4,3-c]isoquinoléine, la 5-(2,3-diméthoxyphényl)-3-méthyl-1H-pyrazol [4,3-c] isoquinoléine, la 5-(2,4-diméthoxyphényl)-3-méthyl-1H-pyrazol[4,3-c]isoquinoléine, la 5-(2,6-diméthoxyphényl)-3-méthyl-1H-pyrazol[4,3-c]isoquinoléine, la 5-(3,4-diméthoxyphényl)-3-méthyl-1H-pyrazol[4,3-c]isoquinoléine, la 5-(3,5-diméthoxyphényl)-3-méthyl-1H-pyrazol[4,3-c]isoquinoléine, la 5-(2,3,4-triméthoxyphényl)-3-méthyl-1H-pyrazol[4,3-c]isoquinoléine, la 5-(2,4,6-triméthoxyphényl)-3-méthyl-1H-pyrazol[4,3-c]isoquinoléine, la 5-(3,4,5-triméthoxyphényl)-3-méthyl-1H-pyrazol[4,3-c]isoquinoléine, la 5-(2-éthoxyphényl)-3-méthyl-1H-pyrazol[4,3-c] isoquinoléine, la 5-(4-diéthylaminophényl)-3-méthyl-1H-pyrazol[4,3-c]isoquinoléine, la 3-méthyl-5-pyridin-4-yl-1H-pyrazol[4,3-c]isoquinoléine, la 3-méthyl-5-pyridin-3-yl-1H-pyrazol[4,3-c]isoquinoléine, la 3-méthyl-5-pyridin-2-yl-1H-pyrazol[4,3-c]isoquinoléine, la 3-méthyl-5-(l-méthylpipéridin-4-yl)-1H-pyrazol[4,3-c]isoquinoléine, la 7,8-diméthoxy-5-(3-méthoxyphényl)-3-méthyl-1H-pyrazol[4,3-c]isoquinoléine, la 7,8-diméthoxy-3-méthyl-5-pyridin-2-yl-1H-pyrazol[4,3-c]isoquinoléine, la 7,8-diméthoxy-3-méthyl-5-pyridin-3-yl-1H-pyrazol[4,3-c]isoquinoléine, la 7-méthoxy-5-(3-méthoxyphényl)-3-méthyl-1H-pyrazol[4,3-c]isoquinoléine, le 2-(3-méthyl-1H-pyrazol[4,3-c]isoquinoléin-5-yl)-phénol, le 4-(3-méthyl-1H-pyrazol[4,3-c]isoquinoléin-5-yl)-benzène-2,4-diol ou le 4-(3-méthyl-1H-pyrazol[4,3-c]isoquinoléin-5-yl)-benzène-1,2-diol.

4. Procédé pour la préparation du composé de formule I selon l'une ou plusieurs des revendications 1 à 3, **caractérisé en ce qu'**on transforme
a) un composé de formule IV 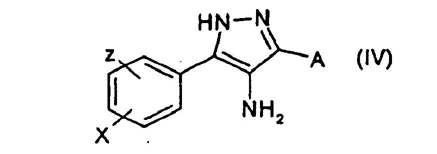 avec un composé des formules Va ou Vb 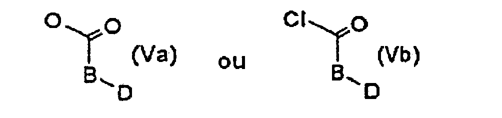 en un composé de formule VI 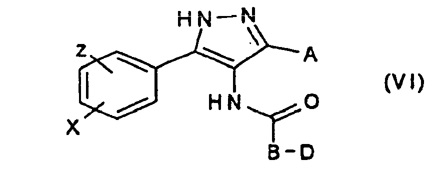 et en présence de pentoxyde de phosphore et d'oxychlorure de phosphore en un composé protégé de formule I puis on dissocie le groupe de protection, où X, Z, A, B et D ont la signification mentionnée dans la revendication 1,
b) on sépare un composé de formule I, préparé selon le procédé a), qui, en raison de sa structure chimique, apparaît sous des formes énantiomères, par salification avec des acides ou des bases sous forme d'énantiomères purs, chromatographie sur des phases stationnaires chirales ou transformation en dérivés au moyen de composés sous forme d'énantiomères purs, tels que des aminoacides, séparation des diastéréo-isomères ainsi obtenus et élimination des groupes auxiliaires chiraux, en énantiomères purs, ou
c) le composé de formule I, préparé selon les procédés
a) ou b) est soit isolé sous forme libre, soit, dans le cas de la présence de groupes acides ou basiques, converti en sels physiologiquement acceptables.

5. Médicament, **caractérisé par** une teneur active en au moins un composé de formule I selon l'une ou plusieurs des revendications 1 à 3 avec un support, un additif et/ou d'autres substances actives et adjuvants pharmaceutiquement approprié(s) et physiologiquement compatible(s).

6. Utilisation du composé de formule II 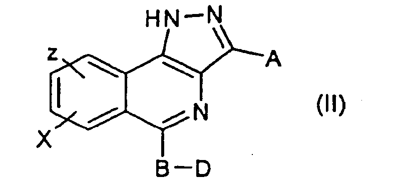 et/ou d'une forme stéréo-isomère du composé de formule II et/ou d'un sel physiologiquement acceptable du composé de formule II, où
A représente
1. -(C₁-C₆)-alkyle, où alkyle est linéaire ou ramifié et est monosubstitué ou disubstitué, indépendamment l'un de l'autre, par
1.1 -O-R1 ou
1.2 C(O)-OR1, où R1 représente
a) un atome d'hydrogène ou
b) - (C₁-C₆)-alkyle,
2. -O-R1
3. C (O) -OR1,
4. hétéroaryle comprenant 5 à 14 chaînons de cycle, où hétéroaryle est non substitué ou substitué,
5. -(C₁-C₆)-alkyle, où alkyle est linéaire ou ramifié, ou
6. -(C₆-C₁₄)-aryle, où aryle est non substitué ou monosubstitué, disubstitué ou trisubstitué, indépendamment l'un de l'autre, par R2,
B représente
1. une liaison covalente ou
2. -(C₁-C₄)-alkylène, où alkylène est linéaire ou ramifié et est monosubstitué ou disubstitué, indépendamment l'un de l'autre, par R1 et R1 est défini comme ci-dessus,
D représente
1. hétéroaryle comprenant 5 à 14 chaînons de cycle, où hétéroaryle est non substitué ou monosubstitué, disubstitué ou trisubstitué, indépendamment l'un de l'autre, par R2, où R2 représente
a) un atome d'hydrogène
b) -(C₁-C₄)-alkyle,
c) -OH,
d) -O- (C₁-C₄) -alkyle,
e) halogène ou
f) -N(R3)-R4, où R3 et R4 signifient, indépendamment l'un de l'autre, un atome d'hydrogène ou -(C₁-C₄)-alkyle,
2. hétérocycle comprenant 5 à 12 chaînons de cycle, où hétérocycle est non substitué ou monosubstitué, disubstitué ou trisubstitué, indépendamment l'un de l'autre, par R2 et R2 est défini comme ci-dessus,
3. (C₆-C₁₄) -aryle, où aryle est non substitué ou monosubstitué, disubstitué ou trisubstitué, indépendamment l'un de l'autre, par R2, et R2 est défini comme ci-dessus, ou
4. -(C₃-C₆)-cycloalkyle, où cycloalkyle est non substitué ou monosubstitué, disubstitué ou trisubstitué, indépendamment l'un de l'autre, par R2, et R2 est défini comme ci-dessus, et
X et Z sont identiques ou différents et représentent, indépendamment l'un de l'autre,
1. un atome d'hydrogène
2. -(C₁-C₄)-alkyle,
3. -OH
4. -O-(C₁-C₄)-alkyle ou
5. halogène
pour la préparation d'un médicament destiné à la prophylaxie et la thérapie de l'ostéoarthrite ou de réactions de rejet du corps contre un organe transféré
ou de réactions de rejet de l'organe transféré contre le corps.

7. Utilisation du composé de formule II selon la revendication 6, où
A représente
1. - (C₁-C₃) -alkyle, où alkyle est linéaire ou ramifié et non substitué ou monosubstitué ou disubstitué, indépendamment l'un de l'autre, par
1.1 -O-R1 ou
1.2 C(O)-OR1, où R1 représente
a) un atome d'hydrogène ou
b) - (C₁-C₃)-alkyle,
2. phényle ou
3. C(O)-OR1,
B représente une liaison covalente
D représente
1. phényle, où phényle est non substitué ou monosubstitué, disubstitué ou trisubstitué, indépendamment l'un de l'autre, par R2, où R2 représente
a) un atome d'hydrogène
b) - (C₁-C₄)-alkyle ou
c) -N(R3)-R4, où R3 et R4 signifient, indépendamment l'un de l'autre, un atome d'hydrogène ou -(C₁-C₃)-alkyle, ou
2. pyridyle, où pyridyle est non substitué ou monosubstitué, disubstitué ou trisubstitué, indépendamment l'un de l'autre, par R2, et R2 est défini comme ci-dessus, ou
3. -(C₄-C₆)-cycloalkyle, où cycloalkyle est non substitué ou monosubstitué, disubstitué ou trisubstitué, indépendamment l'un de l'autre, par R2, et R2 est défini comme ci-dessus, et
X et Z sont identiques ou différents et représentent, indépendamment l'un de l'autre, un atome d'hydrogène ou halogène.
